# EUROPEAN PATENT APPLICATION

(11) **EP 3 266 870 A1**
(43) Date of publication of application: **10.01.2018**
(21) Application number: 16759036.3
(22) Date of filing: 04.03.2016
(51) Int. Cl.: C12N 15/09, A61K 39/395, A61K 45/00, A61K 47/14, A61K 47/42, A61K 47/48, A61P 3/00, A61P 31/04, A61P 31/12, A61P 35/00, B01J 20/24, C07K 14/715, C07K 16/00, C07K 19/00, G01N 33/53

(54) **CONJUGATE AND USE THEREOF**

(30) Priority: 05.03.2015 JP 2015043459
(71) Applicant: The University of Tokyo, Bunkyo-ku, Tokyo 113-8654 (JP); Kanagawa Prefectural Hospital Organization, Yokohama-shi, Kanagawa 2310005 (JP)
(72) Inventor: IMAI, Kohzoh, Tokyo 113-8654 (JP); TSUJI, Shotaro, Yokohama-shi Kanagawa 241-8515 (JP)
(74) Representative: Desaix, Anne
(86) International application number: PCT/JP2016/056797
(87) International publication number: WO 2016/140344

(57) **Abstract**

A technique is provided which enables simple and low-cost purification of biologically active proteins by means of a conjugate of a target substance capturing molecule and a protein which comprises the amino acid sequence in SEQ ID NO:1, or a protein which comprises the amino acid sequence obtained by deleting, substituting or adding one or multiple amino acids in the amino acid sequence in SEQ ID NO: 1 and which has binding activity to a compound having -OH or -OR¹ [R¹ represents a hydrogen atom, an alkyl group or -PO₃H₂].

SEQUENCE LISTING

<110>  THE UNIVERSITY OF TOKYO
       KANAGAWA PREFECTURAL HOSPITAL ORGANIZATION

<120>  A CONNECTED BODY AND USE THEREOF

<130>  PC-21597

<150>  JP2015-043459
<151>  2015-03-05

<160>  10

<170>  PatentIn version 3.5

<210>  1
<211>  313
<212>  PRT
<213>  Homo sapiens

<220>
<221>  misc_feature
<223>  human intelectin-1

<400>  1

Met Asn Gln Leu Ser Phe Leu Leu Phe Leu Ile Ala Thr Thr Arg Gly
1               5                   10                  15

Trp Ser Thr Asp Glu Ala Asn Thr Tyr Phe Lys Glu Trp Thr Cys Ser
            20                  25                  30

Ser Ser Pro Ser Leu Pro Arg Ser Cys Lys Glu Ile Lys Asp Glu Cys
        35                  40                  45

Pro Ser Ala Phe Asp Gly Leu Tyr Phe Leu Arg Thr Glu Asn Gly Val
    50                  55                  60

Ile Tyr Gln Thr Phe Cys Asp Met Thr Ser Gly Gly Gly Gly Trp Thr
65                  70                  75                  80

Leu Val Ala Ser Val His Glu Asn Asp Met Arg Gly Lys Cys Thr Val
                85                  90                  95

Gly Asp Arg Trp Ser Ser Gln Gln Gly Ser Lys Ala Asp Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Tyr Asp Ile Gln Ala
    130                 135                 140

Lys Asp Leu Gly Ile Trp His Val Pro Asn Lys Ser Pro Met Gln His
145                 150                 155                 160

Trp Arg Asn Ser Ser Leu Leu Arg Tyr Arg Thr Asp Thr Gly Phe Leu
                165                 170                 175

Gln Thr Leu Gly His Asn Leu Phe Gly Ile Tyr Gln Lys Tyr Pro Val
            180                 185                 190

Lys Tyr Gly Glu Gly Lys Cys Trp Thr Asp Asn Gly Pro Val Ile Pro
        195                 200                 205

Val Val Tyr Asp Phe Gly Asp Ala Gln Lys Thr Ala Ser Tyr Tyr Ser
    210                 215                 220

Pro Tyr Gly Gln Arg Glu Phe Thr Ala Gly Phe Val Gln Phe Arg Val
225                 230                 235                 240

Phe Asn Asn Glu Arg Ala Ala Asn Ala Leu Cys Ala Gly Met Arg Val
                245                 250                 255

Thr Gly Cys Asn Thr Glu His His Cys Ile Gly Gly Gly Gly Tyr Phe
            260                 265                 270

Pro Glu Ala Ser Pro Gln Gln Cys Gly Asp Phe Ser Gly Phe Asp Trp
        275                 280                 285

Ser Gly Tyr Gly Thr His Val Gly Tyr Ser Ser Ser Arg Glu Ile Thr
    290                 295                 300

Glu Ala Ala Val Leu Leu Phe Tyr Arg
305                 310

<210>  2
<211>  942
<212>  DNA
<213>  Homo sapiens

<220>
<221>  misc_feature
<223>  human intelectin-1

<400>  2
atgaaccaac tcagcttcct gctgtttctc atagcgacca ccagaggatg gagtacagat      60

gaggctaata cttacttcaa ggaatggacc tgttcttcgt ctccatctct gcccagaagc     120

tgcaaggaaa tcaaagacga atgtcctagt gcatttgatg gcctgtattt tctccgcact     180

gagaatggtg ttatctacca gaccttctgt gacatgacct ctgggggtgg cggctggacc     240

ctggtggcca gcgtgcatga gaatgacatg cgtgggaagt gcacggtggg cgatcgctgg 300

tccagtcagc agggcagcaa agcagactac ccagaggggg acggcaactg ggccaactac 360

aacacctttg gatctgcaga ggcggccacg agcgatgact acaagaaccc tggctactac 420

gacatccagg ccaaggacct gggcatctgg cacgtgccca ataagtcccc catgcagcac 480

tggagaaaca gctccctgct gaggtaccgc acggacactg gcttcctcca gacactggga 540

cataatctgt ttggcatcta ccagaaatat ccagtgaaat atggagaagg aaagtgttgg 600

actgacaacg gcccggtgat ccctgtggtc tatgattttg gcgacgccca gaaaacagca 660

tcttattact caccctatgg ccagcgggaa ttcactgcgg gatttgttca gttcagggta 720

tttaataacg agagagcagc caacgccttg tgtgctggaa tgagggtcac cggatgtaac 780

actgagcacc actgcattgg tggaggagga tactttccag aggccagtcc ccagcagtgt 840

ggagattttt ctggttttga ttggagtgga tatggaactc atgttggtta cagcagcagc 900

cgtgagataa ctgaggcagc tgtgcttcta ttctatcgtt ga 942

<210> 3
<211> 891
<212> DNA
<213> Homo sapiens

<220>
<221> misc_feature
<223> human intelectin-1 S165A variant

<400> 3
ggtaccgatg aggctaatac ttacttcaag gaatggacct gttcttcgtc tccatctctg 60

cccagaagct gcaaggaaat caaagacgaa tgtcctagtg catttgatgg cctgtatttt 120

ctccgcactg agaatggtgt tatctaccag accttctgtg acatgacctc tgggggtggc 180

ggctggaccc tggtggccag cgtgcatgag aatgacatgc gtgggaagtg cacggtgggc 240

gatcgctggt ccagtcagca gggcagcaaa gcagactacc cagaggggga cggcaactgg 300

gccaactaca acacctttgg atctgcagag gcggccacga gcgatgacta caagaaccct 360

ggctactacg acatccaggc caaggacctg ggcatctggc acgtgcccaa taagtccccc 420

atgcagcact ggagaaacag cgccctgctg aggtatcgca cggacactgg cttcctccag 480

acactgggac ataatctgtt tggcatctac cagaaatatc cagtgaaata tggagaagga 540

aagtgttgga ctgacaacgg cccggtgatc cctgtggtct atgatttggg cgacgcccag 600

aaaacagcat cttattactc accctatggc cagcgggaat tcactgcggg atttgttcag 660

ttcagggtat ttaataacga gagagcagcc aacgccttgt gtgctggaat gagggtcacc 720

ggatgtaaca ctgagcacca ctgcattggt ggaggaggat actttccaga ggccagtccc 780

cagcagtgtg gagatttttc tggttttgat tggagtggat atggaactca tgttggttac 840

agcagcagcc gtgagataac tgaggcagct gtgcttctat tctatcgttg a 891

<210> 4
<211> 675
<212> DNA
<213> Homo sapiens

<220>
<221> misc_feature
<223> MH166 mAb heavy chain Vh-CH1 region

<400> 4
gaggtccagc tgcaacaatc tggacctgag ctggtgaagc ctggggcttc agtgaagatg 60

tcctgtaagg cgtctggata cacattcact gactactaca tgaagtgggt gaagcagagt 120

catggaaaga gccttgagtg gattggagaa attaatccta aaagtggggg ttctaggtac 180

aaccagaagt tcaagggcaa ggccacaatg actgtagaca aatcctccag cacagcctac 240

atgcagctca acagcctgac atctgaggac tctgcagtct actactgtgc aagagtgggt 300

tactacgccg agggctatgc tatggactac tggggtcaag gaacctcagt caccgtctcc 360

tcagccaaaa cgacaccccc atctgtctat ccactggccc ctggatctgc tgcccaaact 420

aactccatgg tgaccctggg atgcctggtc aagggctatt tccctgagcc agtgacagtg 480

acctggaact ctggatccct gtccagcggt gtgcacacct tcccagctgt cctgcagtct 540

gacctctaca ctctgagcag ctcagtgact gtcccctcca gcacctggcc cagcgagacc 600

gtcacctgca acgttgccca cccggccagc agcaccaagg tggacaagaa aattgtgccc 660

agggattgtg gtacc 675

<210> 5
<211> 657
<212> DNA
<213> Homo sapiens

<220>
<221> misc_feature
<223> MH166 mAb light chain

<400> 5
gagctcgtga tgacccagtc tccagcttct ttggctgtgt ctctagggca gagggccacc 60

atctcctgca gagccagcga aagtgttgat agttatggca ttagttttat gaactggttc 120

caacagaaac caggacagcc acccaaactc ctcatctatg cagcatccaa ccaagtatcc 180

ggggtccctg ccaggtttag tggcagtggg tctgggacag acttcagcct caacatccat 240

cctatggagg aggacgatac tgcaatgtat ttctgtcagc aaagtaagga ggttccgtac 300

acgttcggag gggggaccaa gctggaaata aaacgggctg atgctgcacc aactgtatcc 360

atcttcccac catccagtga gcagttaaca tctggaggtg cctcagtcgt gtgcttcttg 420

aacaacttct accccaaaga catcaatgtc aagtggaaga ttgatggcag tgaacgacaa 480

aatggcgtcc tgaacagttg gactgatcag gacagcaaag acagcaccta cagcatgagc 540

agcaccctca cgttgaccaa ggacgagtat gaacgacata acagctatac ctgtgaggcc 600

actcacaaga catcaacttc acccattgtc aagagcttca acaggaatga gtgttag 657

<210> 6
<211> 777
<212> DNA
<213> Homo sapiens

<220>
<221> misc_feature
<223> human TNFR2

<400> 6
atggcaccag tagctgtttg ggcagcgctg gccgtcggcc tggagctctg ggcggctgct 60

cacgccttgc ccgcccaggt ggcctttacg ccttatgctc cagaacccgg atctacatgt 120

cgccttcgag aatactacga tcagacggcg cagatgtgct gcagcaagtg ctctcccgga 180

caacatgcca aggtattctg cacaaagacc agtgacactg tgtgcgatag ctgtgaggac 240

tctacttaca cgcagctgtg gaattgggtc ccagagtgtc tgtcctgcgg ctcacgctgt 300

agctctgacc aggttgagac acaggcctgt acacgggagc agaaccggat ctgtacatgc 360

agacctggat ggtattgcgc tctttccaaa caagaggggt gtaggctgtg tgcacctctg 420

agaaaatgcc gtccaggctt cggggttgca aggcctggca ctgaaactag tgacgtggtg 480

tgcaaaccat gtgctcccgg tacattttcc aacaccacct catcaaccga tatatgcaga 540

ccgcaccaga tctgcaatgt ggtcgccatt cccggtaacg catccatgga cgccgtctgt 600

accagcacca gccccacaag gtcaatggcc cctggagcag tgcatctccc tcaacctgtg 660

agtacccgga gccagcacac ccagccaact ccggaaccct ccactgctcc tagcactagt 720

ttcttgctgc ccatgggccc ctccccaccg gccgaagggt ctaccgggga tggtacc 777

<210> 7
<211> 313
<212> PRT
<213> Mus musculus

<220>
<221> misc_feature
<223> mouse intelectin-1

<400> 7

Met Thr Gln Leu Gly Phe Leu Leu Phe Ile Met Val Ala Thr Arg Gly

1               5                   10                  15

Cys Ser Ala Ala Glu Glu Asn Leu Asp Thr Asn Arg Trp Gly Asn Ser
            20                  25                  30

Phe Phe Ser Ser Leu Pro Arg Ser Cys Lys Glu Ile Lys Gln Glu His
        35                  40                  45

Thr Lys Ala Gln Asp Gly Leu Tyr Phe Leu Arg Thr Lys Asn Gly Val
    50                  55                  60

Ile Tyr Gln Thr Phe Cys Asp Met Thr Thr Ala Gly Gly Gly Trp Thr
65                  70                  75                  80

Leu Val Ala Ser Val His Glu Asn Asn Met Arg Gly Lys Cys Thr Val
                85                  90                  95

Gly Asp Arg Trp Ser Ser Gln Gln Gly Asn Arg Ala Asp Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Phe Asp Ile Gln Ala
    130                 135                 140

Glu Asn Leu Gly Ile Trp His Val Pro Asn Lys Ser Pro Leu His Asn
145                 150                 155                 160

Trp Arg Lys Ser Ser Leu Leu Arg Tyr Arg Thr Phe Thr Gly Phe Leu
                165                 170                 175

Gln His Leu Gly His Asn Leu Phe Gly Leu Tyr Lys Lys Tyr Pro Val
            180                 185                 190

Lys Tyr Gly Glu Gly Lys Cys Trp Thr Asp Asn Gly Pro Ala Leu Pro
        195                 200                 205

Val Val Tyr Asp Phe Gly Asp Ala Arg Lys Thr Ala Ser Tyr Tyr Ser
    210                 215                 220

Pro Ser Gly Gln Arg Glu Phe Thr Ala Gly Tyr Val Gln Phe Arg Val
225                 230                 235                 240

Phe Asn Asn Glu Arg Ala Ala Ser Ala Leu Cys Ala Gly Val Arg Val
                245                 250                 255

Thr Gly Cys Asn Thr Glu His His Cys Ile Gly Gly Gly Gly Phe Phe
            260                 265                 270

Pro Glu Gly Asn Pro Val Gln Cys Gly Asp Phe Ala Ser Phe Asp Trp
        275                 280                 285

Asp Gly Tyr Gly Thr His Asn Gly Tyr Ser Ser Ser Arg Lys Ile Thr
    290                 295                 300

Glu Ala Ala Val Leu Leu Phe Tyr Arg
305                 310

<210>  8
<211>  942
<212>  DNA
<213>  Mus musculus

<220>
<221>  misc_feature
<223>  mouse intelectin-1

<400>  8
atgacccaac tgggattcct gctgtttatc atggttgcta ccagaggttg cagtgcagct      60

gaagagaacc tggacaccaa caggtggggc aattctttct tttcctctct gcccagaagc     120

tgcaaggaaa tcaagcagga gcacacaaag gcacaagatg gtctctattt cctgcgcacg     180

aagaatggtg tcatctacca gaccttctgt gacatgacca ctgcaggtgg tggctggacc     240

ctggtggcta gtgtgcatga gaacaacatg cgtgggaagt gcactgtggg tgatcgctgg     300

tccagtcagc aaggcaacag agctgactac ccagaggggg atggcaactg ggccaactac     360

aacacctttg ggtctgcaga ggctgccaca agtgatgact acaagaaccc tggctacttt     420

gacatccagg ctgagaacct gggcatctgg cacgtgccca acaaaagccc cctgcacaac     480

tggaggaaga gctccctgct gaggtaccgc accttcactg gcttcctgca gcacttggga     540

cataatctgt ttggcctcta caagaagtac ccagtgaaat acggagaagg aaagtgttgg     600

actgacaatg gcccagcatt acctgtggtc tatgactttg gtgatgctcg gaagacagcc     660

tcttattact ccccctctgg ccagagggaa tttactgcag gatatgttca gttcagagtg     720

tttaataatg agagagcggc cagtgccttg tgtgctggcg tgagggtcac tggatgtaat     780

actgaacatc actgcatcgg tggaggagga ttcttcccag aaggtaaccc cgtgcagtgt     840

ggagactttg cgtcatttga ttgggatgga tatggaactc acaatgggta cagcagtagc     900

cggaagataa ctgaagcagc cgtgcttctg ttttatcgct ga                        942

<210>  9
<211>  313
<212>  PRT

<213>  Homo sapiens

<220>
<221>  misc_feature
<223>  human intelectin-1 C31S, C48S variant

<400>  9

Met Asn Gln Leu Ser Phe Leu Leu Phe Leu Ile Ala Thr Thr Arg Gly
1               5                   10                  15

Trp Ser Thr Asp Glu Ala Asn Thr Tyr Phe Lys Glu Trp Thr Ser Ser
            20                  25                  30

Ser Ser Pro Ser Leu Pro Arg Ser Cys Lys Glu Ile Lys Asp Glu Ser
        35                  40                  45

Pro Ser Ala Phe Asp Gly Leu Tyr Phe Leu Arg Thr Glu Asn Gly Val
    50                  55                  60

Ile Tyr Gln Thr Phe Cys Asp Met Thr Ser Gly Gly Gly Gly Trp Thr
65                  70                  75                  80

Leu Val Ala Ser Val His Glu Asn Asp Met Arg Gly Lys Cys Thr Val
                85                  90                  95

Gly Asp Arg Trp Ser Ser Gln Gln Gly Ser Lys Ala Asp Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Tyr Asp Ile Gln Ala
    130                 135                 140

Lys Asp Leu Gly Ile Trp His Val Pro Asn Lys Ser Pro Met Gln His
145                 150                 155                 160

Trp Arg Asn Ser Ser Leu Leu Arg Tyr Arg Thr Asp Thr Gly Phe Leu
                165                 170                 175

Gln Thr Leu Gly His Asn Leu Phe Gly Ile Tyr Gln Lys Tyr Pro Val
            180                 185                 190

Lys Tyr Gly Glu Gly Lys Cys Trp Thr Asp Asn Gly Pro Val Ile Pro
        195                 200                 205

Val Val Tyr Asp Phe Gly Asp Ala Gln Lys Thr Ala Ser Tyr Tyr Ser
    210                 215                 220

Pro Tyr Gly Gln Arg Glu Phe Thr Ala Gly Phe Val Gln Phe Arg Val
225                 230                 235                 240

Phe Asn Asn Glu Arg Ala Ala Asn Ala Leu Cys Ala Gly Met Arg Val
                245                 250                 255

Thr Gly Cys Asn Thr Glu His His Cys Ile Gly Gly Gly Gly Tyr Phe
            260                 265                 270

Pro Glu Ala Ser Pro Gln Gln Cys Gly Asp Phe Ser Gly Phe Asp Trp
        275                 280                 285

Ser Gly Tyr Gly Thr His Val Gly Tyr Ser Ser Ser Arg Glu Ile Thr
    290                 295                 300

Glu Ala Ala Val Leu Leu Phe Tyr Arg
305                 310

<210>  10
<211>  942
<212>  DNA
<213>  Homo sapiens

<220>
<221>  misc_feature
<223>  human intelectin-1 C31S, C48S variant

<400>  10
atgaaccaac tcagcttcct gctgtttctc atagcgacca ccagaggatg gagtacagat      60

gaggctaata cttacttcaa ggaatggacc tcttcttcgt ctccatctct gcccagaagc     120

tgcaaggaaa tcaaagacga atctcctagt gcatttgatg gcctgtattt tctccgcact     180

gagaatggtg ttatctacca gaccttctgt gacatgacct ctgggggtgg cggctggacc     240

ctggtggcca gcgtgcatga gaatgacatg cgtgggaagt gcacggtggg cgatcgctgg     300

tccagtcagc agggcagcaa agcagactac ccagaggggg acggcaactg ggccaactac     360

aacacctttg gatctgcaga ggcggccacg agcgatgact acaagaaccc tggctactac     420

gacatccagg ccaaggacct gggcatctgg cacgtgccca ataagtcccc catgcagcac     480

tggagaaaca gctccctgct gaggtaccgc acggacactg gcttcctcca gacactggga     540

cataatctgt ttggcatcta ccagaaatat ccagtgaaat atggagaagg aaagtgttgg     600

actgacaacg gcccggtgat ccctgtggtc tatgattttg gcgacgccca gaaaacagca     660

tcttattact caccctatgg ccagcgggaa ttcactgcgg gatttgttca gttcagggta     720

tttaataacg agagagcagc caacgccttg tgtgctggaa tgagggtcac cggatgtaac     780

actgagcacc actgcattgg tggaggagga tactttccag aggccagtcc ccagcagtgt 840

ggagattttt ctggttttga ttggagtgga tatggaactc atgttggtta cagcagcagc 900

cgtgagataa ctgaggcagc tgtgcttcta ttctatcgtt ga 942

## Description

### TECHNICAL FIELD

The present invention relates to a conjugate and the use thereof. The present application claims priority based on Japanese Patent Application No. 2015-043459 filed to Japan Patent Office on March 5, 2015, the content of which is incorporated herein.

### BACKGROUND ART

Antibody pharmaceuticals are purified during the manufacturing process using Protein A bound resins that bind to the Fc region (for example, see Patent Document 1). Protein A bound resins are expensive, and the usability after washing is limited. Thus, despite the various improvements that have been achieved, the cost for antibody purification has not dropped significantly, which is part of the reason for the high price of antibody pharmaceuticals.

Furthermore, since antibody pharmaceuticals are highly humanized, there is almost no difference from the antibodies that exist abundantly within the body, making it impossible to specifically adsorb and remove administered antibody pharmaceuticals by a plasma purification device or the like.

### PRIOR ART DOCUMENTS

### Patent Documents

Patent Document 1: Japanese Examined Patent Application Publication No. Sho 61-29932

### SUMMARY OF THE INVENTION

### Problems to Be Solved by the Invention

The present invention is intended to provide a technology that allows feasible purification of a biologically active protein, for example an antibody, at low cost. The present invention is further intended to provide a conjugate, a vector, a pharmaceutical composition, a pharmaceutical, a method of purifying a conjugate, a solid phase for capturing a conjugate, a method of removing a target substance, a device for removing a target substance, a labeling compound for detecting a conjugate, a method of detecting a conjugate, a conjugate for detecting a compound, and a method of detecting a compound.

### Means for Solving the Problem

The present invention comprises the aspects below.
(1) A conjugate of:
   a protein consisting of the amino acid sequence of SEQ ID NO: 1, or
   a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂.]; and
   a functional molecule.
(2) The conjugate according to (1), wherein said functional molecule is a target substance capturing molecule.
(3) The conjugate according to (2), wherein said target substance capturing molecule is an antibody, an antibody fragment, an aptamer, or a receptor against said target substance.
(4) The conjugate according to (2) or (3), wherein said target substance capturing molecule is tumor necrosis factor receptor (TNFR).
(5) The conjugate according to any one of (2) - (4), wherein said target substance is a cytokine.
(6) A vector characterized by containing a nucleic acid encoding a fusion protein of:
   a protein consisting of the amino acid sequence of SEQ ID NO: 1, or
   a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂.]; and
   an antibody, an antibody fragment, a peptide aptamer, or a receptor against a target substance.
(7) A pharmaceutical composition characterized by comprising:
   a drug;
   a carrier containing a compound comprising -OH and -OR¹ [R¹ represents a hydrogen atom, an alkyl group, or -PO₃H₂.]; and
   a conjugate according to any one of (1) - (5) bound to said compound.
(8) A pharmaceutical characterized by containing a conjugate according to any one of (1) - (5) as an active ingredient.
(9) A method of purifying a conjugate, the method characterized by comprising
   contacting a conjugate according to any one of (1) - (5) with a compound comprising -OH and -OR¹ [R¹ represents a hydrogen atom, an alkyl group, or - PO₃H₂.] fixed to a solid phase.
(10) A solid phase for capturing a conjugate according to any one of (1) - (5), the solid phase characterized in that a compound comprising -OH and -OR¹ [R¹ represents a hydrogen atom, an alkyl group, or -PO₃H₂.] is fixed to said solid phase.
(11) A method of removing a conjugate according to any one of (1) - (5), the method characterized by comprising
   contacting plasma containing said conjugate with a compound comprising - OH and -OR¹ [R¹ represents a hydrogen atom, an alkyl group, or -PO₃H₂.] fixed to a solid phase.
(12) A method of removing a target substance, the method characterized by comprising contacting
   plasma containing a complex of a target substance and a conjugate according to any one of (2) - (5) with
   a compound comprising -OH and -OR¹ [R¹ represents a hydrogen atom, an alkyl group, or -PO₃H₂.] fixed to a solid phase.
(13) A method of removing a target substance, the method characterized by comprising contacting
   plasma containing a target substance with
   a conjugate according to any one of (2) - (5) fixed to a solid phase.
(14) A device for removing a conjugate according to any one of (1) - (5), the device characterized by comprising
   a removing means for removing said conjugate from plasma containing said conjugate, wherein said removing means comprises a solid phase to which a compound comprising -OH and -OR¹ [R¹ represents a hydrogen atom, an alkyl group, or -PO₃H₂.] is fixed.
(15) A device for removing a target substance, characterized by comprising
   a removing means for removing a complex of a target substance and a conjugate according to any one of (2) - (5) from plasma containing said complex, wherein said removing means comprises a solid phase to which a compound comprising -OH and -OR¹ [R¹ represents a hydrogen atom, an alkyl group, or - PO₃H₂.] is fixed.
(16) A device for removing a target substance, the device characterized by comprising
   a removing means for removing a target substance from plasma containing said target substance, wherein said removing means comprises a solid phase to which a conjugate according to any one of (2) - (5) is fixed.
(17) A labeling compound for detecting a conjugate according to any one of (1) - (5), the compound characterized by comprising -OH and -OR¹ [R¹ represents a hydrogen atom, an alkyl group, or -PO₃H₂.].
(18) A method of detecting a conjugate, the method comprising:
   forming a complex by allowing a labeling compound comprising -OH and - OR¹ [R¹ represents a hydrogen atom, an alkyl group, or -PO₃H₂.] to bind to a conjugate according to any one of (1) - (5); and
   detecting the labeling compound in said complex.
(19) A conjugate for detecting a compound comprising -OH and -OR¹ [R¹ represents a hydrogen atom, an alkyl group, or -PO₃H₂.], the conjugate characterized by being a conjugate of:
   a protein consisting of the amino acid sequence of SEQ ID NO: 1, or
   a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂.]; and
   a labeling substance.
(20) A method of detecting a compound comprising -OH and -OR¹ [R¹ represents a hydrogen atom, an alkyl group, or -PO₃H₂.], the method characterized by comprising:
   forming a complex by allowing a conjugate for detecting a compound according to (19) to bind to a compound comprising -OH and -OR¹ [R¹ represents a hydrogen atom, an alkyl group, or -PO₃H₂.]; and
   detecting said labeling substance in said complex.

### Effects of the Invention

According to the present invention, we are able to provide a technology that allows feasible purification of a biologically active protein, for example an antibody, at low cost. We are also able to provide a conjugate, a vector, a pharmaceutical composition, a pharmaceutical, a method of purifying a conjugate, a solid phase for capturing a conjugate, a method of removing a target substance, a device for removing a target substance, a labeling compound for detecting a conjugate, a method of detecting a conjugate, a conjugate for detecting a compound, and a method of detecting a compound.

### BRIEF DESCRIPTION OF THE DRAWINGS

Figure 1 is a schematic diagram describing one embodiment of a pharmaceutical composition.
Figure 2 is a schematic diagram describing one embodiment of a method of removing a target substance and a device for removing a target substance.
Figure 3 is a schematic diagram describing one embodiment of a method of removing a target substance and a device for removing a target substance.
Figure 4 is a schematic diagram describing one embodiment of a labeling compound for detecting a conjugate.
Figure 5 is a schematic diagram describing one embodiment of a conjugate for detecting a compound.
Figure 6A is a photograph that shows a result from Example 1.
Figure 6B is a diagram that shows the structure of individual beads used in Example 1.
Figure 7A is a graph that shows a result from Example 2.
Figure 7B is a graph that shows a result from Example 2.
Figure 7C is a graph that shows a result from Example 2.
Figure 8 is a graph that shows a result from Example 3.
Figure 9 is a graph that shows a result from Example 4.
Figure 10A is a photograph that shows a result from Example 5.
Figure 10B shows general formulae showing the structure of 1-monoacylglycerol, 2-monoacylglycerol, and lysophosphatidic acid.
Figure 11A is a graph that shows a result from Example 7.
Figure 11B is a photograph that shows a result from Example 7.
Figure 12 is a graph that shows a result from Example 8.
Figure 13A is a graph that shows a result from Example 9.
Figure 13B is a graph that shows a result from Example 9.
Figure 14A is a photograph that shows a result from Example 10.
Figure 14B is a photograph that shows a result from Example 10.
Figure 15A is a graph that shows a result from Example 11.
Figure 15B is a graph that shows a result from Example 11.
Figure 16 is a graph that shows a result from Example 12.
Figure 17 is a graph that shows a result from Example 13.
Figure 18 is a graph that shows a result from Example 14.

### DETAILED DESCRIPTION OF THE EMBODIMENTS

The embodiments of the present invention will be described below in detail, while referring to the drawings as needed. In the drawings, a same reference numeral is assigned to identical or corresponding parts, and overlapping explanations are omitted. The dimension ratio in individual drawings may be partially exaggerated for clarification and does not necessarily match the actual ratio.

### [Conjugate]

Inventors found that intelectin (the amino acid sequence of human intelectin-1 is shown in SEQ ID NO: 1.), a kind of lectin, specifically binds to a compound having a particular structure represented by the diol structures described below, and completed the present invention.

In one embodiment, the present invention provides a conjugate of:
a protein consisting of the amino acid sequence of SEQ ID NO: 1, or
a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂.]; and
a functional molecule. Here, when R¹ is a hydrogen atom, the above-mentioned compound is a compound having two -OH groups. Examples of such compounds are, for instance, diols, and details are described later herein.

Here, "one or several" refers to, for example 1-60, for example 1-55, for example 1-50, for example 1-40, for example 1-30, for example 1-20, for example 1-10. The above-mentioned protein may be referred to as "intelectin or the mutant thereof" herein. When R¹ is an alkyl group, the number of carbons of said alkyl group is, for example 1-6, or for example 1-3.

As shown in the Examples described later herein, Inventors found that mouse intelectin-1 also has binding activity to compounds comprising -OH and -OR¹ [R¹ represents a hydrogen atom, an alkyl group, or -PO₃H₂.]. There are 51 amino acids that are different between human intelectin-1 and mouse intelectin-1. Therefore, the intelectin or the mutant thereof within the conjugate of the present embodiment may contain at least 51 mutations in comparison to the amino acid sequence of SEQ ID NO: 1. The amino acid sequence and the nucleotide sequence of mouse intelectin-1 are shown in SEQ ID NO: 7 and SEQ ID NO: 8, respectively.

Wild type human intelectin-1 forms a trimer, but the mutant (C31, 48S), in which each of the cysteine residues at position 31 and position 48 is substituted with a serine residue, does not form a trimer and is produced as a monomer (see Tsuji S., et al., Differential structure and activity between human and mouse intelectin-1: Human intelectin-1 is a disulfide-linked trimer, whereas mouse homologue is a monomer, Glycobiology 17(10), 1045-1051, 2007).

As shown in the Examples described later herein, Inventors found that, as with human intelectin-1, the C31, 48S mutant of human intelectin-1 has binding activity to compounds comprising -OH and -OR¹ [R¹ represents a hydrogen atom, an alkyl group, or -PO₃H₂.].

Therefore, intelectin or the mutant thereof may be produced as a monomer or produced as a multimer, for example a trimer.

As shown in the Examples described later herein, the above-mentioned compound is preferably a compound described as Formula (1) below.

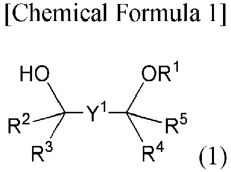

In Formula (1), R¹ represents a hydrogen atom, an alkyl group in which the number of carbons is 1-3, or -PO₃H₂. Y¹ is a single bond or a methylene group. Each of R² - R⁵ independently represents a hydrogen atom or an organic group that may be substituted. When any of R² - R⁵ is an organic group, said organic group may from a branch or a ring, and may comprise an ester bond, an ether bond, or the like. The substituent may be, for example, an alkyl group, a hydroxyl group, an acyl group, a phosphate group, a phosphoric acid ester group, a carboxyl group, an amino group, a nitro group, a thiol group, a sulfo group, a carbonyl group, an acetamide group, a benzoyl group, an alkylene group, an alkynyl group, an aryl group, a silicon compound, a halogen atom, or the like.

As shown in Examples, intelectin or the mutant thereof has high adsorption activity (binding activity) to the compounds described as Formula (1) shown above. Considering the higher binding activity of intelectin or the mutant thereof, R¹ - R⁴ of the compound described as Formula (1) shown above are preferably hydrogen atoms. When R¹ - R⁴ of the compound described as Formula (1) shown above are hydrogen atoms, the structure of R⁵ tends not to have much of an effect on the binding activity of intelectin or the mutant thereof. When R¹ of the compound described as Formula (1) shown above is an hydrogen atom, either R² or R³ is an alkyl group, and either R⁴ or R⁵ is an alkyl group (for example, when a compound comprises a 2,3-diol structure), considering the higher binding activity of intelectin or the mutant thereof, the meso form is more preferable over the cis form or the trans form.

More specific examples of the compound described as Formula (1) are, for instance, saccharides such as L-ribose, D-ribose, 2-deoxygalactose, 2-acetamido-2-deoxy-4-O-beta-D-galactopyranosyl-D-glucopyranose (Galp-GlcNAc), 2-acetamido-2-deoxy-4-O-beta-D-galactofuranosyl-D-glycopyranose (Galf-GlcNAc), 2-deoxyribose, D-xylose, L-xylose, N-acetylneuraminic acid, D-fructose, D-galactose, L-lyxose, D-lyxose, L-sorbose, D-mannose, D-glucose, L-arabinose, D-arabinose, L-rhamnose, N-acetylglucosamine, and D-fucose; saccharic acids such as ascorbic acid; polyols such as monobutyrin, glycerol, 1,2-butanediol, 1,2-propanediol, (R)-1,2-propanediol, (S)-1,2-propanediol, 3-amino-1,2-propanediol, ribitol, glycerophosphoric acid, meso-2,3-butanediol, (R,R)-2,3-butanediol, (S,S)-2,3-butanediol, ethylene glycol, propylene glycol, 1,3-propanediol, and 1,3-butanediol; Tris; monoacylglycerols; and the like.

The compound described as Formula (1) shown above may form a polymer in which multiple compounds are bound to each other. Such a polymer may be, for example, the polymer described as Formula (2) shown below, and polyvinyl alcohols.

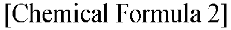

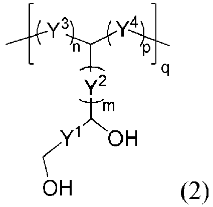

In Formula (2), Y¹ represents the same as Y¹ in Formula (1) shown above, each of Y² - Y⁴ independently represents a bivalent linking group, each of m, n, and p independently represents an integer of 1 - 10, and q is an integer of 2 or larger. The bivalent linking group may be, for example, -CH2-, -O-, -S-, -NH-, -CO-, -CONH-, or -CH(COOH)-. The maximum value of q is, for example, approximately 5000.

The compound described as Formula (2) shown above may be, for example, polyethylene glycol having a diol structure introduced in its side chain, gamma-polyglutamic acid having a diol structure introduced in its side chain, or the like.

### (Functional Molecule)

The functional molecule in a conjugate of the present embodiment is not necessarily restricted to a particular molecule as long as the molecule has a desired function, and may be, a therapeutic pharmaceutical, a diagnostic pharmaceutical, or the like. The above-mentioned pharmaceutical may be, for example, a high molecular weight compound such as a protein, or a low molecular weight compound. More specific examples of a functional molecule is, for example, target substance capturing molecules; pharmaceutical compositions containing a compound conjugated with an anti-cancer agent, a cytotoxic agent, a therapeutic radioisotope (for example, a radio isotope such as P³², Y⁹⁰, I¹³¹, I¹²⁵, Sm¹⁵³, Re¹⁸⁶, Re¹⁸⁸, At²¹¹, Bi²¹², Pb²¹², or Lu), or the like; contrast agents used for computed tomography (CT), magnetic resonance imaging (MRI), or the like; probes for positron emission tomography (PET); and the like. As disclosed later herein, according to the conjugate of the present embodiment, for example, a functional molecule may be collected as needed, after the functional molecule is administered to the body. By enabling the collection of a drug that is a functional molecule, we can expect various effects, such as attempting to reduce a side effect and delaying the timing of autoantibody production associates with, for example, a protein formulation such as antibodies. The conjugate may be used not only in medical applications but also in the research reagent field such as a research tool. In such cases as well, the functional molecule may be a high molecular weight compound such as a protein and a nucleic acid, or a low molecular weight compound, that may be used for a reagent.

### (Target Substance Capturing Molecule)

The target substance capturing molecule in the conjugate of the present embodiment may be, for example, an antibody, an antibody fragment, an aptamer, or the like against said target substance.

When the target substance capturing molecule is an antibody or an antibody fragment intended for administration to a human, the antibody or the antibody fragment is preferably humanized. The antibody fragment may be Fv, Fab, scFv, or the like. The antibody or the antibody fragment may be derived from an antibody pharmaceutical. Since antibody pharmaceuticals are highly humanized, there is almost no difference from the antibodies that exist within the body, and therefore are not be able be removed once administered to a patient. In contrast, the conjugate of the present embodiment may be removed from the patient's body, as needed, by using a target substance removing device or the like described later herein.

Aptamers are substances having a specific binding ability to a target substance. An aptamer may be, for example, a nucleic acid aptamer, a peptide aptamer, or the like. A nucleic acid aptamer having a specific binding ability to a target substance may be sorted, for example, by the systematic evolution of ligand by exponential enrichment (SELEX) method or the like. A peptide aptamer having a specific binding activity to a target substance may, for example, be sorted by the two-hybrid method using yeasts, or the like.

When the target substance capturing molecule is a protein such as an antibody, an antibody fragment, a peptide aptamer, or a receptor, the conjugate of the present embodiment may be a fusion protein with the target substance capturing molecule. When the target substance capturing molecule is a non-protein substance such as a nucleic acid aptamer, the conjugate of the present embodiment may be obtained by, for example, crosslinking intelectin or the mutant thereof and the target substance capturing molecule using a chemical linker or the like.

When the target substance capturing molecule is a protein, such as an antibody, an antibody fragment, or an aptamer against the target substance, or a receptor protein that binds to a cell membrane antigen, the conjugate may be utilized for an antibody pharmaceutical, a drug delivery systems to deliver a drug to a cell expressing the target substance, a probe for detecting the presence of the target substance, collection of the target substance, or the like. Details are described later herein.

The above-mentioned target substance may be, for example, a cancer antigen, for example a cell membrane antigen, for example a cytokine, for example any optional protein that is a research subject (for instance in basic research), or the like. The cancer antigen is not restricted to a particular antigen, and may be, for example, HER2, MAGE, CEA, WT1, KL-6, or the like. The cell membrane antigen may be, for example, CD3, CTLA-4, PD-1, or the like. The cytokine may be, for instance, an inflammatory cytokine. Specifically, the cytokine may be, for instance, TNF-alpha, TNF-beta, LT-beta, interleukin (IL)-1 beta, IL-6, or the like.

When the target substance capturing molecule is a receptor, the receptor may be, for instance, a cytokine receptor, a receptor that bind to a cell membrane antigen, or the like. The cytokines may be, for instance, tumor necrosis factor receptor (TNFR), IL-1 receptor, IL-6 receptor, TRAIL receptor, or the like. TNFR1 and TNFR2, for instance, are TNFRs, but TNFR2 is preferable, considering its higher affinity for TNF. When the conjugate of the present embodiment is a cytokine receptor, the conjugate may be used, for example, for removing cytokines in the blood or for a similar application. Details are described later herein.

As described above, intelectin or the mutant thereof may be produced as a monomer, or produced as a multimer such as a trimer. In general, a multimer has an increased affinity for the target substance than a monomer. Therefore, when a higher affinity for the target substance is desired, intelectin or the mutant thereof may be produced as a multimer. When used as, for example, a purification tag, intelectin or the mutant thereof may be produced as a monomer.

Human intelectin is a plasma protein and does not induce physiological reactions regardless of the concentration in the body fluid. Therefore, the conjugate of the present embodiment is presumed to be less likely to show harmful side effects, even when administered as a pharmaceutical to a human or a non-human animal.

### [Vector]

In one embodiment, the present invention provides a vector characterized by containing a nucleic acid encoding a fusion protein of: intelectin or the mutant thereof; and an antibody, an antibody fragment, a peptide aptamer, or a receptor against a target protein. A nucleic acid encoding intelectin or the mutant thereof may be, for instance: a nucleic acid comprising the nucleotide sequence as set forth in SEQ ID NO: 2; a nucleic acid comprising the nucleotide sequence encoding a protein that has for example 80% or higher, for example 85% or higher, for example 90% or higher, or for example 95% or higher identity to the nucleotide sequence as set forth in SEQ ID NO: 2 and has binding activity to a compound comprising -OH and -OR¹ [R¹ represents a hydrogen atom, an alkyl group, or -PO₃H₂.]; or the like. The nucleotide sequence as set forth in SEQ ID NO: 2 is the nucleotide sequence of human intelectin-1.

The vector of the present embodiment is preferably an expression vector. The vector is not restricted to a particular vector, and, for instance, a plasmid derived from E.coli, such as pBR322, pBR325, pUC12, and pUC13; a plasmid derived from Bacillus subtilis, such as pUB110, pTP5, and pC194; a plasmid derived from yeast, such as pSH 19 and pSH 15; a bacteriophage, such as lambda phage; a viruse, such as an adenovirus, an adeno-associated virus, a lentivirus, a vaccinia virus, and a baculovirus; or a modified vector thereof may be used.

In the above-mentioned expression vector, the promoter for expressing a conjugate is not restricted to a particular promoter, and, for instance, EF1 alpha promoter, SR alpha promoter, SV40 promoter, LTR promoter, CMV (cytomegalovirus) promoter, or HSV-tk promoter may be used.

The above-mentioned expression vector may further comprise an enhancer, a splicing signal, a polyadenylation signal, a selection marker, replication origin, and/or the like.

### [Pharmaceutical Composition]

In one embodiment, the present invention provides a pharmaceutical composition characterized by comprising: a drug; a carrier containing a compound comprising -OH and -OR¹ [R¹ represents a hydrogen atom, an alkyl group, or - PO₃H₂.]; and an above-described conjugate bound to said compound. The pharmaceutical composition of the present embodiment may be a drug delivery system. The above-mentioned compound is preferably the compound described as Formula (1) or (2) shown above.

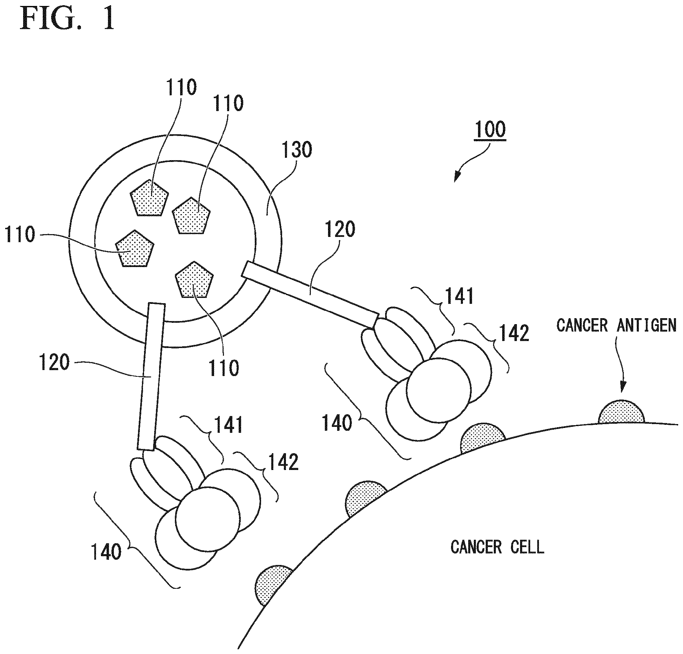

Figure 1 is a schematic diagram describing the structure of the pharmaceutical composition of the present embodiment. In Figure 1, a pharmaceutical composition 100 comprises: a carrier 130 containing a drug 110 and a compound 120; and a conjugate 140 bound to the compound 120. Preferably, the compound 120 is exposed on the surface of the carrier 130.

The conjugate 140 is a conjugate of: intelectin or the mutant thereof 141 as described above; and, for example, an antibody 142 against a cancer antigen. The carrier 130 is not restricted to a particular carrier as long as the carrier can be used for a drug delivery system. The carrier may be, for instance, a micelle, a liposome, or the like.

The micelle is not restricted to a particular micelle as long as the micelle can be used for a drug delivery system, and for example, a micelle formed from a block copolymer comprising a hydrophilic region and a hydrophobic region may be used. The liposome is not restricted to a particular liposome as long as the liposome can be used for a drug delivery system, and for example, a liposome formed from phospholipid may be used.

When manufacturing the drug delivery system of the present embodiment, the compound 120 is added to the ingredients for such micelle or liposome, and a micelle or a liposome is formed. The compound 120 is, for instance, monoacylglycerols such as 1-monoolein, or the like.

The drug 110 is, for instance, an anti-cancer drug such as paclitaxel, docetaxel, vincristine, vinorelbine, vinblastine, vindesine, eribulin mesylate, oxaliplatin, cisplatin, or carboplatin. For example, the drug 110 may be encapsulated in a micelle or a liposome, by forming the micelle or the liposome in the presence of the drug 110.

When exposed to the compound 120, the conjugate 140 easily binds to the compound 120. Therefore, the pharmaceutical composition 100 may be easily manufactured by combining: the carrier 130 containing the compound 120; and the conjugate 140. The pharmaceutical composition 100 of the present embodiment is able to deliver the drug 110 specifically to a tissue or a cell expressing the cancer antigen. Hence, the pharmaceutical composition 100 is able to exert a cytotoxic activity in a cancer cell specific manner, leading to reduced side effects.

### (Modification example)

The pharmaceutical composition of the present invention not only specifically delivers an anti-cancer drug, targeting a cancer cell as described above, but also may be utilized for: specifically delivering an anti-bacterial drug or an anti-viral drug, targeting, for example, a cell infected with bacteria or viruses; specifically delivering a metabolism modulating substance such as an enzyme, an siRNA, a peptide aptamer, a nucleic acid aptamer, a nucleic acid (gene), or the like, targeting an appropriate cell according to the purpose; specifically delivering a labeling substance such as a radioisotope, a fluorescent dye, a fluorescent protein, or the like, targeting an appropriate cell according to the purpose; or a similar application.

In one embodiment, the present invention provides a method of treating or diagnosing cancer or an infectious disease. The method comprises a step of administering to a mammalian animal a pharmaceutical composition comprising:
a cancer drug, an anti-bacterial agent, an anti-viral agent, a metabolism modulating substance, or a labeling substance;
a carrier containing a compound comprising -OH and -OR¹ [R¹ represents a hydrogen atom, an alkyl group, or -PO₃H₂.]; and
a conjugate bound to said compound, wherein the conjugate is a conjugate of an antibody, an antibody fragment, or an aptamer against a cancer antigen or a desired antigen, and
intelectin or the mutant thereof.

In one embodiment, the present invention provides a pharmaceutical composition for treating cancer or a infectious disease, wherein the pharmaceutical composition comprises:
a cancer drug, an anti-bacterial agent, an anti-viral agent, a metabolism modulating substance, or a labeling substance;
a carrier containing a compound comprising -OH and -OR¹ [R¹ represents a hydrogen atom, an alkyl group, or -PO₃H₂.]; and
a conjugate bound to said compound, wherein the conjugate is a conjugate of
   an antibody, an antibody fragment, or an aptamer against a cancer antigen or a desired antigen, and
   intelectin or the mutant thereof.

In one embodiment, the present invention provides a use of a conjugate for manufacturing a pharmaceutical composition for treating cancer or a pharmaceutical composition for treating an infectious disease, wherein the conjugate is a conjugate of: an antibody, an antibody fragment, or an aptamer against a cancer antigen or a desired antigen; and intelectin or the mutant thereof.

### [Pharmaceutical]

In one embodiment, the present invention provides a pharmaceutical containing an above-described conjugate as an active ingredient.

The pharmaceutical of the present invention may be for example an antibody pharmaceutical, or for example an anti-hypercytokinemia therapeutic agent (anti-cytokine drug). The conjugate may be, for instance, an antibody against a target substance, a conjugate of an antibody or an aptamer and intelectin or the mutant thereof, a conjugate of a cytokine receptor and intelectin or the mutant thereof; or the like. The target substance, the cytokine, and the cytokine receptor are as described above.

The pharmaceutical of the present embodiment is applicable to an antibody pharmaceutical that already exists. This enables feasible purification of an antibody pharmaceutical at low cost.

When the pharmaceutical of the present embodiment is a anti-cytokine drug, the target disease may be, for instance, systemic inflammatory response syndrome (SIRS) such as sepsis, which is hypercytokinemia; rheumatism; cancer cachexia; psoriasis; or the like. For example, although there is no effective treatment for severe SIR, administering an anti-cytokine drug of the present invention to a severe SIR patient is able to adsorb an inflammatory cytokine in the blood and ameliorate the symptom. Administering an anti-cytokine drug of the present invention to a patient with rheumatism, cancer cachexia, psoriasis, or the like is able to adsorb an inflammatory cytokine in the body and ameliorate the symptom.

In one embodiment, the present invention provides a method of treating cancer, wherein the method comprises a step of administering to a mammalian animal a pharmaceutical comprising a conjugate of: an antibody, an antibody fragment, or an aptamer against a cancer antigen; and intelectin or the mutant thereof.

In one embodiment, the present invention provides a conjugate for treating cancer, wherein the conjugate is a conjugate of: an antibody, an antibody fragment, or an aptamer against a cancer antigen; and intelectin or the mutant thereof.

In one embodiment, the present invention provides a use of a conjugate for manufacturing a pharmaceutical for treating cancer, wherein the conjugate is a conjugate of: an antibody, an antibody fragment, or an aptamer against a cancer antigen; and intelectin or the mutant thereof.

In one embodiment, the present invention provides a method of treating a disease caused by high cytokine production, wherein the method comprises a step of administering to a mammalian animal a pharmaceutical comprising a conjugate of: an antibody, an antibody fragment, or an aptamer, or a cytokine receptor against a cytokine; and intelectin or the mutant thereof.

In one embodiment, the present invention provides a conjugate for treating a disease caused by high cytokine production, wherein the conjugate is a conjugate of: an antibody, an antibody fragment, or an aptamer, or a cytokine receptor against a cytokine; and intelectin or the mutant thereof.

In one embodiment, the present invention provides a use of a conjugate for manufacturing a pharmaceutical for treating a disease caused by high cytokine production, wherein the conjugate is a conjugate of: an antibody, an antibody fragment, or an aptamer, or a cytokine receptor against a cytokine; and intelectin or the mutant thereof.

### [Generically Modified Organism]

In one embodiment, the present invention provides a genetically modified organism expressing a conjugate described above. The genetically modified organism may be, for instance: a cultured cell such as a bacterial, yeast, insect, or animal cell, with an expression vector of the conjugate described above introduced therein; an insect organism, such as a silk worm, with an expression vector of the conjugate described above introduced therein; an animal, such as a goat, sheep, a cow, a chicken, or the like, that is genetically modified to express the conjugate described above in the milk or the egg; or the like.

According to the genetically modified organism of the present invention, a conjugate described may be manufactured. The manufactured conjugate may be easily purified at low cost using a method described later herein.

### [Method of Purifying a Conjugate]

In one embodiment, the present invention provides a method of purifying a conjugate, wherein the method is characterized by comprising a step of contacting the conjugate described above to a compound comprising -OH and -OR¹ [R¹ represents a hydrogen atom, an alkyl group, or -PO₃H₂.] fixed to a solid phase. The above-mentioned compound is preferably the compound described as Formula (1) or (2) shown above. The solid phase to which the above-mentioned compound (solid phase for capturing a conjugate) is fixed is described later herein.

Herein, the term "purification" may also be referred to as "separation," "collection," "adsorption," "binding," or the like. According to the purification method of the present embodiment, a conjugate expressed in the above-mentioned genetically modified organism culture, insect body, milk, egg, or the like, may be easily purified at low cost.

Specifically, first, a conjugate expressed by a genetically modified organism described above, contained in the culture, insect body, milk, or egg, and a solid phase to which a compound comprising -OH and -OR¹ [R¹ represents a hydrogen atom, an alkyl group, or -PO₃H₂.] is fixed are contacted. The intelectin or the mutant thereof region of the conjugate is then adsorbed to the above-mentioned compound, thereby allowing the solid phase to adsorb the conjugate.

Subsequently, the conjugate adsorbed to the solid phase is washed with buffer or the like. The conjugate adsorbed to the solid phase is then brought in contact with, for example, buffer or the like containing a compound comprising free -OH and -OR¹ [R¹ represents a hydrogen atom, an alkyl group, or -PO₃H₂.].This separates the conjugate from the solid phase, enabling collection of the conjugate. The above-mentioned compound in its free state may be, for instance, glycerol, propylene glycol, ribose, or the like.

Inventors also discovered that the conjugate of intelectin or the derivative thereof and a compound comprising -OH and -OR¹ [R¹ represents a hydrogen atom, an alkyl group, or -PO₃H₂.] is Ca²⁺ ion, Sr²⁺ ion, or Mn²⁺ ion dependent, and that the conjugate may be separated from a solid phase and collected by contacting buffer or the like containing ethylenediaminetetraacetic acid (EDTA) to the above-mentioned conjugate adsorbed to the above-mentioned solid phase.

As described later herein, the solid phase to which a compound comprising - OH and -OR¹ [R¹ represents a hydrogen atom, an alkyl group, or -PO₃H₂.] is fixed is much less expensive compared to, for example, Protein A bound resins, and is tolerant to denaturing wash treatments. Therefore, when the conjugate is a conjugate of intelectin or the mutant thereof and an antibody that is an antibody therapeutic, the purification method of the present embodiment allows for substantial cost reduction in manufacturing antibody therapeutics and enables inexpensive provision of antibody therapeutics.

Furthermore, there are compounds with very high safety and stability among compounds comprising -OH and -OR¹ [R¹ represents a hydrogen atom, an alkyl group, or -PO₃H₂.], represented by diols. For example, propylene glycol is a compound used as injection solvent, and the safety and stability are very high. Therefore, a conjugate purified by the purification method of the present embodiment using such a compound has high stability even when used as pharmaceuticals.

### [Solid Phase for Capturing a Conjugate]

In one embodiment, the present invention provides a solid phase for capturing a conjugate described above, wherein the solid phase is characterized by being fixed to a compound comprising -OH and -OR¹ [R¹ represents a hydrogen atom, an alkyl group, or -PO₃H₂.]. The above-mentioned compound is preferably a compound described as Formula (1) or (2) shown above. A solid phase may be, for instance, a bead, a membrane, a hollow fiber membrane, a plastic base plate, a well plate, a glass slide, a sensor chip for measuring equipments such as surface plasmon resonance measuring devices, or the like The solid phase in the form of a bead, a membrane, or the like may be packed in a column.

Compound comprising -OH and -OR¹ [R¹ represents a hydrogen atom, an alkyl group, or -PO₃H₂.], represented by diols, is able to be optionally introduced on the surface of a resin or the like in a highly inexpensive manner. For example, by treating an excess amount of diepoxide, such as ethylene glycol glycidyl ether, 1,4-butanediol diglycidyl ether, or the like and performing alkaline hydrolysis or acid hydrolysis, an above-mentioned compound may be easily fixed to a solid phase comprising a hydroxyl group, an amino group, or a thiol group. An above-mentioned compound may be fixed onto a solid phase comprising a carboxy group by conjugating the solid phase with 3-amino-1,2-propanediol via 1-ethyl-3-[3-dimethylaminopropyl]carbodiimide hydrochloride (EDC). Furthermore, an above-mentioned compound may be fixed onto a solid phase comprising an amino group by conjugating the solid phase with glyceric acid via EDC.

The reusability of Protein A bound resins, for example, is limited, due to some reasons such as deterioration of Protein A cause by washes and sterilization. Furthermore, when a Protein A bound resin is used to purify an antibody, following denaturing and eluting the antibody with acidic buffer, procedures such as neutralizing the eluted antibody and then purifying the antibody further using an ion-exchange column are required, and there are many necessary steps for purifying antibodies.

In contrast, the compound comprising -OH and -OR¹ [R¹ represents a hydrogen atom, an alkyl group, or -PO₃H₂.] hardly does not deteriorate, even with an acid, an alkali, high pressure steam sterilization or the like is performed. Therefore, with the solid phase for capturing a conjugate of the present embodiment, almost all washing or sterilizing methods that the solid phase can tolerate may be performed. Furthermore, such a compound binds to a binding agent (intelectin) at a high binding rate, provides high purification purity, and gives high recovery efficiency of 95% or above. Since the toxicity and the protein denaturing effect of the eluent such as glycerol, propylene glycol, or ribose are low, such eluent allows a conjugate eluted from the solid phase for capturing a conjugate of the present embodiment to be adsorbed by an ion-exchange column, enabling feasible conjugate purification.

### [Method of Removing a Target Substance]

### (First embodiment)

In one embodiment, the present invention provides a method of removing a target substance. The method is characterized by comprising a step of contacting plasma containing a complex of a target substance and an above-mentioned conjugate to a compound comprising -OH and -OR¹ [R¹ represents a hydrogen atom, an alkyl group, or -PO₃H₂.] fixed to a solid phase. The above-mentioned compound is preferably a compound described as Formula (1) or (2) shown above. The method of removing a target substance of the present embodiment may be also referred to as a method of removing a target substance from plasma, or a method of removing a conjugate from plasma. Furthermore, the method of removing a target substance of the present embodiment may be considered as a kind of apheresis, which is a method of harvesting a desired blood component from whole blood.

The conjugate may be, for instance: an antibody therapeutic that is a conjugate of intelectin or the mutant thereof and an antibody or an antibody fragment; a conjugate of intelectin or the mutant thereof and an antibody, an antibody fragment, or a peptide aptamer against a target substance; a conjugate of intelectin or the mutant thereof and a cytokine receptor; or the like. The target substance mentioned above may be, for instance, a toxin, a pathogenic substance, an inflammatory cytokine, a virus, a bacterium, a prion, or the like. What is obtained by from patient's blood using a membrane plasma separator or the like may be utilized as plasma.

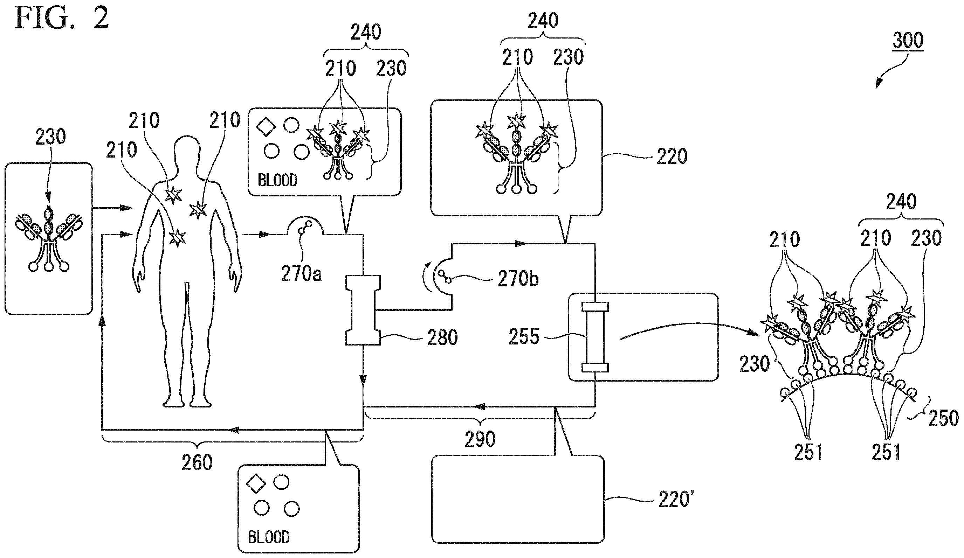

Figure 2 is a schematic diagram describing the first embodiment of a method of removing a target substance. In Figure 2, a conjugate 230 of intelectin or the mutant thereof and an antibody, an antibody fragment, a peptide aptamer, a cytokine receptor, or the like against a target substance is administered to plasma 220 in advance, and a target substance 210 forms a complex 240 with the conjugate 230.

The method of removing a target substance of the present embodiment comprises a step of contacting an above-mentioned solid phase 250 for capturing a target substance to the above-mentioned plasma 220. In Figure 2, a column 255 is packed with the solid phase 250. A compound 251 comprising -OH and -OR¹ [R¹ represents a hydrogen atom, an alkyl group, or -PO₃H₂.] is fixed to the solid phase 250. In this step, when the solid phase 250 comes in contact with the plasma 220, the intelectin or the mutant thereof portion of the conjugate 230 forming the complex 240 binds to the compound 251. As a result, the complex 240 is adsorbed to the solid phase 250, and the target substance 210 is removed from the plasma 220. In Figure 2, the target substance 210 is removed from plasma 220' eluted from the column 255.

The target substance 210 in the plasma 220 may be specifically removed using the method of the present embodiment. The plasma 220' with the target substance 210 removed may be placed back in the patient's body. Therefore, the conjugate 230 may also be considered as a tool for collecting a target substance for apheresis.

### (Second embodiment)

In one embodiment, the present invention provides a method of removing a target substance. The method is characterized by comprising a step of contacting plasma containing a target substance to an above-mentioned conjugate fixed to a solid phase. The method of removing a target substance of the present embodiment may also be considered as a kind of apheresis, which is a method of collecting a desired blood component from whole blood.

A conjugate similar to the above-mentioned conjugate in the first embodiment of the method of removing a target substance as described above may be used as the conjugate.

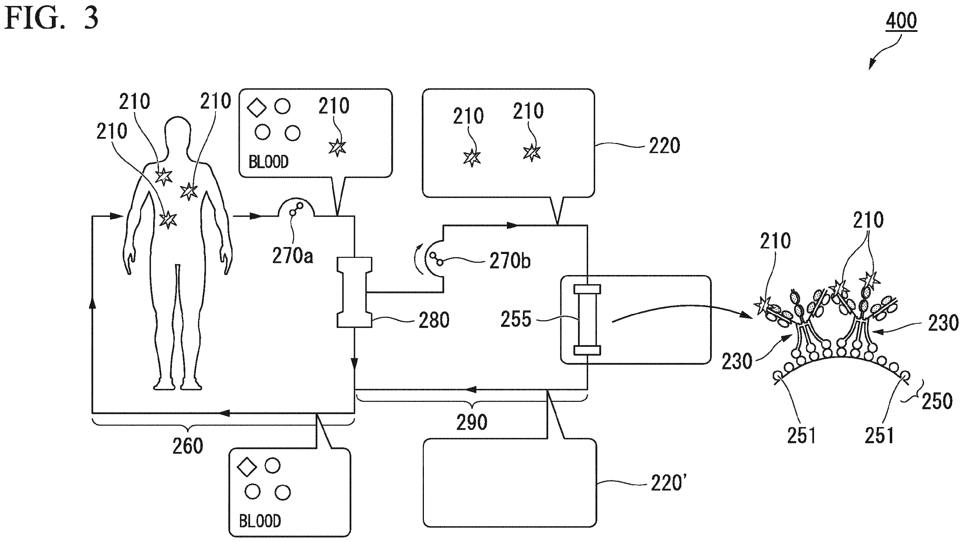

Figure 3 is a schematic diagram describing the second embodiment of the method of removing a target substance. In Figure 3, a column 255 is packed with a solid phase 250 to which a conjugate 230 is fixed. A compound 251 comprising -OH and -OR¹ [R¹ represents a hydrogen atom, an alkyl group, or -PO₃H₂.] is fixed to the solid phase 250. Intelectin or the mutant thereof of the conjugate 230 binds to the compound 251, thereby the conjugate 230 is fixed to the solid phase 250.

When plasma 220 containing a target substance 210 and the conjugate 230 bound to the solid phase 250 are contacted, the conjugate 230 binds to the target substance 210.

As a result, the target substance 210 is adsorbed to the solid phase 250, and the target substance 210 is removed from the plasma 220. In Figure 3, the target substance 210 is removed from the plasma 220' discharged from the column 255.

The target substance 210 in the plasma 220 may be specifically removed by the method of the present embodiment. The plasma 220' with the target substance 210 removed may be placed back in the body of a patient. Therefore, the conjugate 230 may be considered as a tool for collecting a target substance for apheresis, in the present embodiment as well.

Systemic inflammatory response syndrome (SIRS) has no effective therapy. Cytokine-specific plasma adsorption therapy is expected to be able to dramatically improve the treatment outcome, but practical development of the therapy is difficult with existing technologies. The method of the present embodiment is practical from safety and cost perspectives, and may be considered to be a breakthrough treatment for SIRS.

If a conjugate that binds to a cytokine is used as the conjugate 230 in the first embodiment or the second embodiment of the above-mentioned method of removing a target substance, the cytokine in the plasma may be removed. This may be able to prevent multiple organ failure caused by advanced SIRS and dramatically improve the survival rate. Furthermore, the method of the present embodiment may be applied to rheumatism, cancer cachexia, psoriasis, or the like, in which cytokine increase within the body is one of the causes.

### (Third embodiment)

In one embodiment, the present invention provides a method of removing an above-mentioned conjugate. The method is characterized by comprising a step of contacting plasma containing said conjugate to a compound comprising -OH and - OR¹ [R¹ represents a hydrogen atom, an alkyl group, or -PO₃H₂.] fixed to a solid phase. According to the method of the present embodiment, for example, the above-mentioned conjugate administered to the blood of a patient may be removed from the body of the patient.

More specifically, an example is, for instance, administering an above-mentioned conjugate comprising a contrast agent as a functional molecule to a patient and performing an examination by computed tomography (CT), magnetic resonance imaging (MRI), or the like, followed by removing the contrast agent in the blood. This will be able to reduce side effects caused by the contrast agent.

### [Devices for Removing a Target Substance]

### (First embodiment)

In one embodiment, the present invention provides a device for removing a target substance. The device is characterized by comprising a removing means for removing a complex of a target substance and an above-mentioned conjugate from plasma containing said complex, wherein said removing means comprises a solid phase to which a compound comprising -OH and -OR¹ [R¹ represents a hydrogen atom, an alkyl group, or -PO₃H₂.] is fixed. Said compound is preferably a compound described as Formula (1) or (2) shown above.

A conjugate similar to the conjugate in the first embodiment of the method of removing a target substance as described above may be used as the conjugate.

Figure 2 is a schematic diagram describing a device for removing a target substance 300 of the present embodiment. The device for removing a target substance 300 is a device suitable for performing the first embodiment of the method of removing a target substance as described above.

The device for removing a target substance 300 comprises a solid phase for capturing a conjugate (removing means) 250 as described above. The device for removing a target substance 300 may further comprise, a blood circuit 260 for circulating the blood of a patient, a pump 270a for transporting the blood within the blood circuit 260, a plasma separation device 280 for separating the plasma from the blood, a plasma circuit 290 for circulating the plasma 220 separated by the plasma separation device 280, a pump 270b for transporting the plasma within the plasma circuit 290, and/or the like.

A conjugate 230 of intelectin or the mutant thereof and an antibody, an antibody fragment, a peptide aptamer, a cytokine receptor, or the like against a target substance 210 is administered in advance to a patient to whom the device for removing a target substance 300 is applied, and the target substance 210 forms a complex 240 with the conjugate 230 in the blood of the patient.

The blood harvested from the patient circulates within the blood circuit 260 by the pump 270a. The blood will then be separated into the plasma 220 and the blood cell compartment in the plasma separation device 280. The separated plasma 220 circulates within the plasma circuit 290 by the pump 270b. The plasma 220 then comes in contact with the removing means 250.

In Figure 2, a column 255 is packed with the removing means 250. A compound 251 comprising -OH and -OR¹ [R¹ represents a hydrogen atom, an alkyl group, or -PO₃H₂.] is fixed to the removing means 250. When the removing means 250 is contacted to the plasma 220, the intelectin or the mutant thereof portion of the conjugate 230 forming the complex 240 binds to the compound 251. As a result, the complex 240 is adsorbed to the removing means 250, and the target substance 210 is removed from the plasma 220. In Figure 2, the target substance 210 is removed from the plasma 220' discharged from the column 255. The plasma 220' with the target substance 210 removed is returned to the blood circuit 260.

The target substance 210 in the plasma 220 may be specifically removed by the device for removing a target substance of the present embodiment. The plasma 220' with the target substance 210 removed may be returned into the body of the patient.

### (Second embodiment)

In one embodiment, the present embodiment provides a device for removing a target substance. The device is characterized by comprising a removing means for removing a target substance from plasma containing said target substance, wherein said removing means comprises a solid phase to which an above-mentioned conjugate is fixed.

A conjugate similar to the conjugate in the first embodiment of the method of removing a target substance as described above may be used as the conjugate.

Figure 3 is a schematic diagram describing a device for removing a target substance 400 of the present embodiment. The device for removing a target substance 400 is a device suitable for performing the second embodiment of the method of removing a target substance as described above.

The device for removing a target substance 400 comprises an above-mentioned conjugate (removing means) 230 bound to an above-mentioned solid phase 250 for capturing a conjugate. The device for removing a target substance 400 may further comprise, a blood circuit 260 for circulating the blood of a patient, a pump 270a for transporting the blood within the blood circuit 260, a plasma separation device 280 for separating the plasma from the blood, a plasma circuit 290 for circulating the plasma 220 separated by the plasma separation device 280, a pump 270b for transporting the plasma within the plasma circuit 290, and/or the like.

The blood harvested from the patient circulates within the blood circuit 260 by the pump 270a. The blood is then separated into the plasma 220 and the blood cell compartment in the plasma separation device 280. The separated plasma 220 circulates within the plasma circuit 290 by the pump 270b. The plasma 220 then comes in contact with the removing means 230.

In Figure 3, a column 255 is packed with the removing means 230. The removing means 230 is bound to a compound 251 comprising -OH and -OR¹ [R¹ represents a hydrogen atom, an alkyl group, or -PO₃H₂.] fixed to the solid phase 250.

When the plasma 220 containing a target substance 210 and the conjugate 230 bound to the solid phase 250 are allowed to come in contact, the conjugate 230 binds to the target substance 210.

As a result, the target substance 210 is adsorbed to the solid phase 250, and the target substance 210 is removed from the plasma 220. In Figure 3, the target substance 210 is removed from the plasma 220' discharged from the column 255. The plasma 220' with the target substance 210 removed is returned to the blood circuit 260.

The target substance 210 in the plasma 220 may be specifically removed by the device for removing a target substance of the present embodiment. The plasma 220' with the target substance 210 removed may be returned into the body of the patient. In the first embodiment and the second embodiment of the device for removing a target substance as described above, the device for removing a target substance may be used as a cytokine removing device for removing a cytokine in plasma, if a conjugate that binds to a cytokine is used as the conjugate 230. Multiple organ failure caused by advancement of SIRS may be prevented and the survival rate may be dramatically improved by removing a cytokine in the plasma of a patient using the present device. A treatment on rheumatism, cancer cachexia, psoriasis, or the like, in which cytokine increase within the body is one of the causes, may be performed removing a cytokine in the plasma of a patient using the present device.

### (Third embodiment)

In one embodiment, the present invention provides a device for removing an above-mentioned conjugate. The device is characterized by comprising a removing means for removing an above-mentioned conjugate from plasma containing said conjugate, wherein said removing means comprises a solid phase to which a compound comprising -OH and -OR¹ [R¹ represents a hydrogen atom, an alkyl group, or -PO₃H₂.] is fixed. According to the removing device of the present embodiment, for example, the above-mentioned conjugate administered to the blood of a patient may be removed from the patient's body.

### [Labeling Compound for Detecting a Conjugate]

In one embodiment, the present invention provides a labeling compound for detecting an above-mention conjugate, wherein the compound is characterized by comprising -OH and -OR¹ [R¹ represents a hydrogen atom, an alkyl group, or - PO₃H₂.]. Considering its high binding activity to intelectin or the mutant thereof, the labeling compound for detecting a conjugate of the present embodiment is preferably a conjugate of a compound described as Formula (2) shown above and a labeling substance such as a radioisotope, a fluorescent substance, an enzyme, a fluorescent protein, or the like.

Examples of more specific labeling substances are, for instance, alkaline phosphatase, horseradish peroxidase, GFP, biotin, FITC, Cy2, Cy3, Cy5, rhodamines, Alexa Fluor dyes, and the like.

The labeling compound for detecting a conjugate of the present embodiment binds specifically to the intelectin or the mutant thereof portion of the above-mentioned conjugate. Therefore, the presence of a target substance that a conjugate recognizes may be detected by, for example, utilizing a conjugate having an intelectin or the mutant thereof portion and an antibody, antibody fragment, or peptide aptamer portion against the target substance in lieu of a primary antibody, and utilizing a labeling compound for detecting a conjugate of the present embodiment in lieu of a secondary antibody.

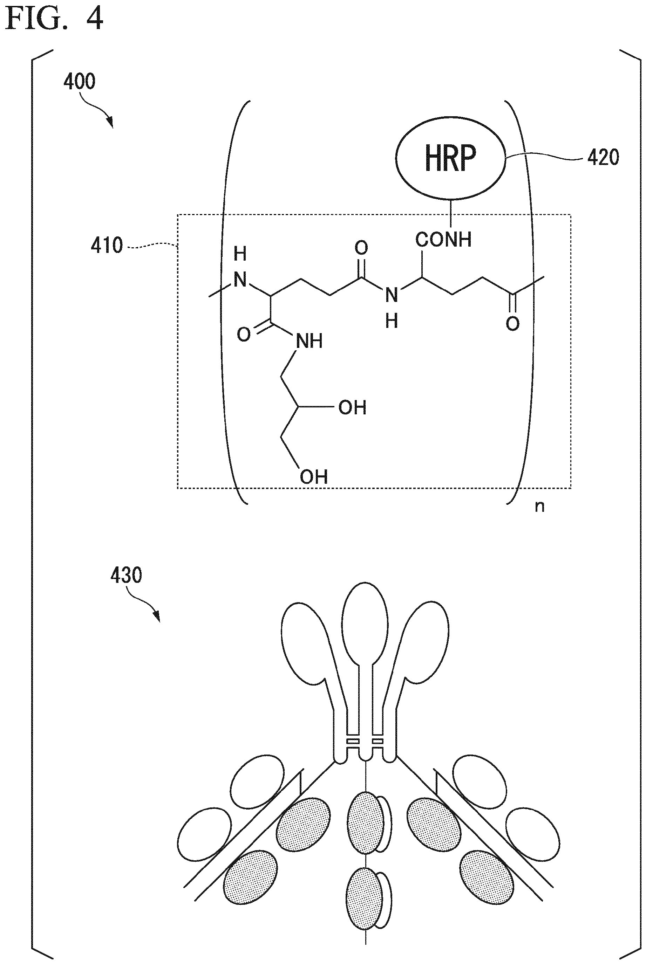

Figure 4 is a schematic diagram describing a labeling compound for detecting a conjugate of the present embodiment. The labeling compound 400 for detecting a conjugate is a conjugate of a compound 410 comprising -OH and -OR¹ [R¹ represents a hydrogen atom, an alkyl group, or -PO₃H₂.] and a labeling substance 420. In Figure 4, the main chain of the compound 410 is gamma-poly glutamic acid, and the labeling compound 420 is horseradish peroxidase.

The labeling compound 400 for detecting a conjugate is, for example, able to bind specifically to the intelectin or the mutant thereof portion of a fusion protein (conjugate) 430 of intelectin and the mutant thereof and an antibody. Therefore, the labeling compound 400 may be utilized in detection of a conjugate. Here, although an antibody is used in the conjugate 430, an aptamer, a receptor, or the like may be used instead of the antibody.

### [Method of Detecting a Conjugate]

In one embodiment, the present invention provides a method of detecting a conjugate. The method is characterized by comprising: a step of allowing the formation of a complex by attaching a labeling compound comprising -OH and -OR¹ [R¹ represents a hydrogen atom, an alkyl group, or -PO₃H₂.] to an above-mentioned conjugate; and a step of detecting the labeling compound of said complex. The above-mentioned labeling compound is preferably a compound described as Formula (2) shown above.

The presence of a target substance that a conjugate recognizes may be detected by the detection method of the present embodiment, for example, using in lieu of a primary antibody a conjugate comprising an intelectin or the mutant thereof portion and an antibody, antibody fragment, or peptide aptamer portion against the target substance, and using in lieu of a secondary antibody a labeling compound for detecting a conjugate as described above.

Detection of a labeling compound for detecting a conjugate may be conducted by using a fluorescent microscope or the like, if the labeling substance within the compound for detecting a conjugate is a fluorescent substance, a fluorescent protein, or the like. When the labeling substance is an enzyme, the detection may be conducted by having the enzyme react with an appropriate substrate and detecting color development, luminescence, fluorescence, or the like.

### [Conjugate for Detecting a Compound]

In one embodiment, the present invention provides a conjugate for detecting a compound comprising -OH and -OR¹ [R¹ represents a hydrogen atom, an alkyl group, or -PO₃H₂.], wherein the conjugate is characterized by being a conjugate of intelectin or the mutant thereof and a labeling substance.

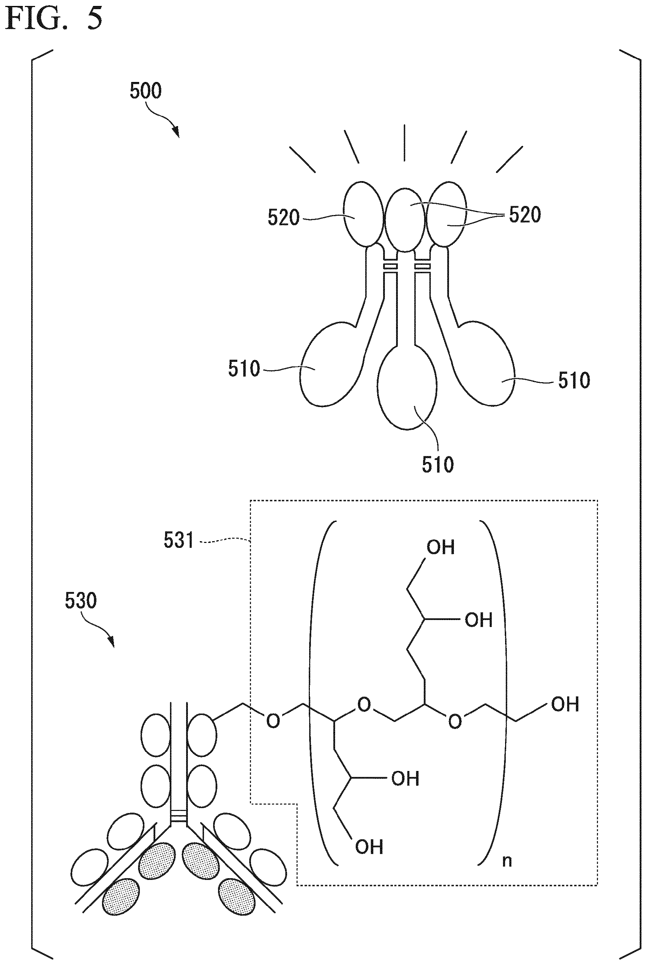

Figure 5 is a schematic diagram describing a conjugate for detecting a compound of the present embodiment. The conjugate for detecting a compound 500 is a conjugate of intelectin or the mutant thereof 510 and a labeling substance 520. A labeling substance similar to the labeling substance in the above-mentioned labeling compound for detecting a conjugate may be used as the labeling substance 520.

The conjugate for detecting a compound 500, with its intelectin or the mutant thereof 510 portion, is able to bind specifically to a compound comprising -OH and - OR¹ [R¹ represents a hydrogen atom, an alkyl group, or -PO₃H₂.], and therefore may be utilized in the detection of said compound.

For example, as shown in Figure 5, the presence of a target substance that an antibody 530 recognizes may be detected, by using the antibody 530 labeled with a compound 531 comprising -OH and -OR¹ [R¹ represents a hydrogen atom, an alkyl group, or -PO₃H₂.] as a primary antibody, and using a conjugate for detecting a compound 500 in lieu of a secondary antibody.

In the example shown in Figure 5, the labeling substance 520 is a fluorescent substance and the compound 531 is polyethylene glycol with a diol structure introduced in the side chain. Although the antibody 530 is shown in Figure 5, an aptamer, a receptor, or the like may be used instead of the antibody.

### [Method of Detecting a Compound]

In one embodiment, the present invention provides a method of detecting a compound comprising -OH and -OR¹ [R¹ represents a hydrogen atom, an alkyl group, or -PO₃H₂.].The method is characterized by comprising: a step of allowing the formation of a complex by attaching an above-mentioned conjugate for detecting a compound to a compound comprising -OH and -OR¹ [R¹ represents a hydrogen atom, an alkyl group, or -PO₃H₂.]; and a step of detecting said labeling substance of said complex. The above-mentioned compound is preferably a compound described as Formula (2) shown above.

The presence of a target substance that the antibody 530 recognizes may be detected by the detecting method of the present embodiment, for example, using the antibody 530 shown in Figure 5 as a primary antibody and using the conjugate for detecting a compound 500 in lieu of a secondary antibody.

The presence of a target substance that an antibody, an antibody fragment, a peptide aptamer, a receptor, or the like recognizes may be detected, using in lieu of a primary antibody an antibody, an antibody fragment, a peptide aptamer, a receptor, or the like labeled with a compound comprising -OH and -OR¹ [R¹ represents a hydrogen atom, an alkyl group, or -PO₃H₂.], and using the above-mentioned conjugate for detecting a compound in lieu of a secondary antibody.

The detection of a labeling substance may be conducted in a similar manner as in the method for detecting a compound described above.

### EXAMPLES

Although the present invention is explained using the Examples below, the present invention is not to be restricted to the following Examples.

### [Example 1]

### (Binding of intelectin and a diol group)

Beads having a diol group on the surface and beads not having a diol group on the surface were prepared. Beads obtained by conjugating 3-amino-1-propanediol bound to the surface of polystyrene beads via an amino group (diol beads), and beads obtained by alkaline hydrolyzing the epoxy group of Sepharose beads with 1,4-bis(2,3-epoxypropyl)butane introduced (epoxy-activated Sepharose 6B (GE Healthcare Biosciences)) (diol Sepharose beads) were used as the beads having a diol group on the surface. Polystyrene beads and beads obtained by conjugating 3-amino-1-propanol to polystyrene beads (hydroxy beads) were used as the beads not having a diol group on the surface.

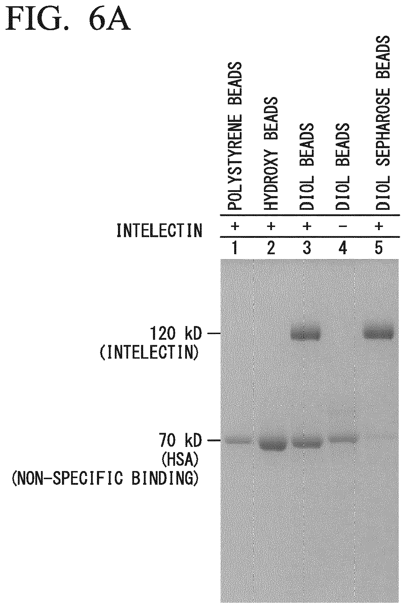

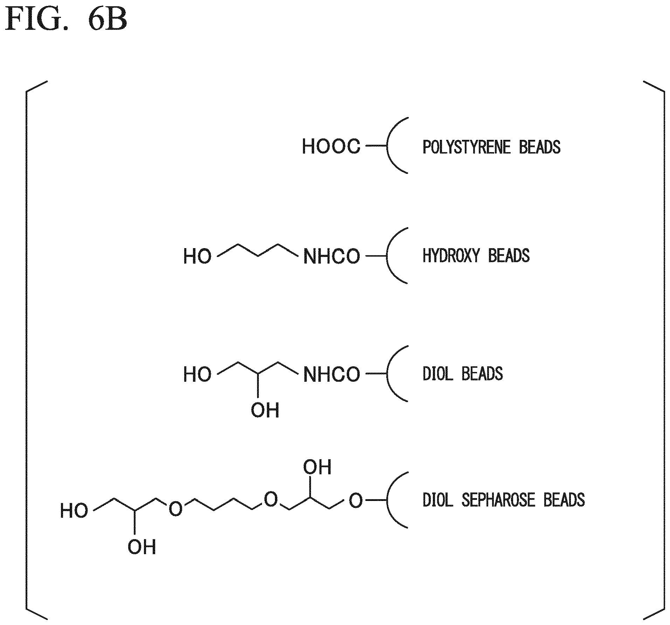

Each kind of beads were added to 500 microliters of a medium containing intelectin or a medium not containing intelectin, and were stirred for 18 hours at 25 deg C. Recombinant human intelectin-1 was used as intelectin. Subsequently, the beads were collected by centrifugation and washed once with buffer, and the samples was eluted with buffer containing 15% glycerol. Samples were separated by SDS polyacrylamide gel electrophoresis (SDS-PAGE), and proteins were stained using Coomassie Brilliant Blue. Figure 6A is a photograph that shows an SDS-PAGE result, and Figure 6B is a diagram that shows the structure of individual beads.

Accordingly, intelectin is now revealed to be able to selectively bind to diol beads and diol Sepharose beads.

Furthermore, similar results were obtained when beads obtained by acid hydrolyzing the epoxy group of Sepharose beads with 1,4-bis(2,3-epoxypropyl)butane introduced (epoxy-activated Sepharose 6B (GE Healthcare Biosciences)) were used as the diol Sepharose beads having a diol group on the surface.

### [Example 2]

### (Inhibition of the binding of intelectin and a diol group by various compounds 1)

7.5 microliters of diol Sepharose beads and 65 microliters of buffer (20 mM Tris buffer (pH 7.0) containing 1 mM CaCl₂, 150 mM NaCl, and 0.1% Tween 20) containing various kinds of compounds that were serially diluted were added to 35 microliters of a medium containing intelectin, and the medium was stirred for 36 hours at 4 deg C. Recombinant human intelectin-1 was used as intelectin. The above-mentioned compound was a compound containing or not containing a diol group. Specifically, the compound was glycerol, 1,2-propanediol, 1,3-propanediol, 1-propanol, ethylene glycol, ethanolamine, ethanol, 1,2-butanediol, 1,3-butanediol, 1,4-butanediol, D-2,3-butanediol, L-2,3-butanediol, meso-2,3-butanediol, 3-amino-1,2-propanediol, 3-amino-1-propanol, galactose, glucose, mannose, or ribose.

Subsequently, the supernatant from each sample was collected by centrifugation, and the concentration of the remaining intelectin in the supernatant was measured by sandwich ELISA using an anti-intelectin monoclonal antibody.

The intelectin concentration measured using the sample without the addition of an above-mentioned compound was considered as 100%, the intelectin concentration measured using a medium not containing intelectin as a sample was considered as 0%, and the inhibition rates on the binding of intelectin and the diol group were calculated.

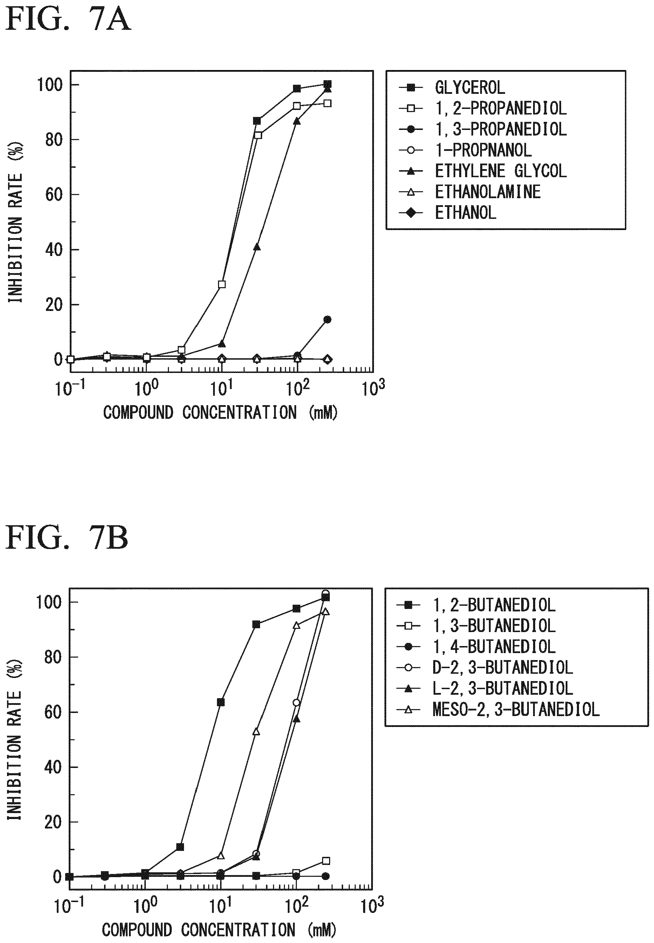

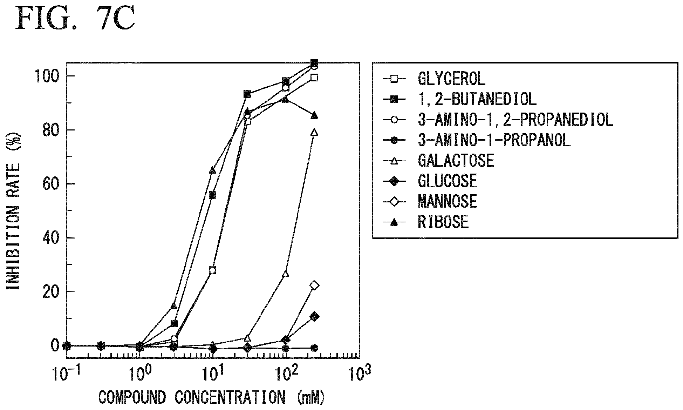

Figures 7A-C are the graphs that show experiment results. Intelectin showed high affinities for compounds comprising a 1,2-diol group. Intelectin also showed low affinities for compounds comprising the 2,3-diol structure.

### [Example 3]

### (Inhibition of the binding of intelectin and a diol group by various compounds 2)

The binding of purified intelectin to a sensor chip to which 3-amino-1-propanediol is fixed via an amino group was measured using Biacore (GE Healthcare Biosciences) under the presence of various kinds of compounds that were serially diluted. Recombinant human intelectin-1 was used as intelectin. The above-mentioned compound was a compound containing or not containing a diol group. Specifically, the compound was glycerol, 1,2-propanediol, 1,3-propanediol, 1-propanol, ethylene glycol, ethanol, 2-methoxyethanol, galactose, 2-acetamido-2-deoxy-4-O-beta-D-galactopyranosyl-D-glucopyranose (Galp-GlcNAc), 2-acetamido-2-deoxy-4-O-beta-D-galactofuranosyl-D-glucopyranose (Galf-GlcNAc), D-ribose, or L-ribose.

Intelectin was used at the final concentration of 0.5 microgram/mL. 10 mM HEPES (pH 7.0) containing 1 mM CaCl₂, 150 mM NaCl, and 0.03% Tween 20 was used as the measuring buffer. The binding of intelectin measured using the sample that was not added with an above-mentioned compound was considered 100%, and the binding of intelectin in each sample was measured.

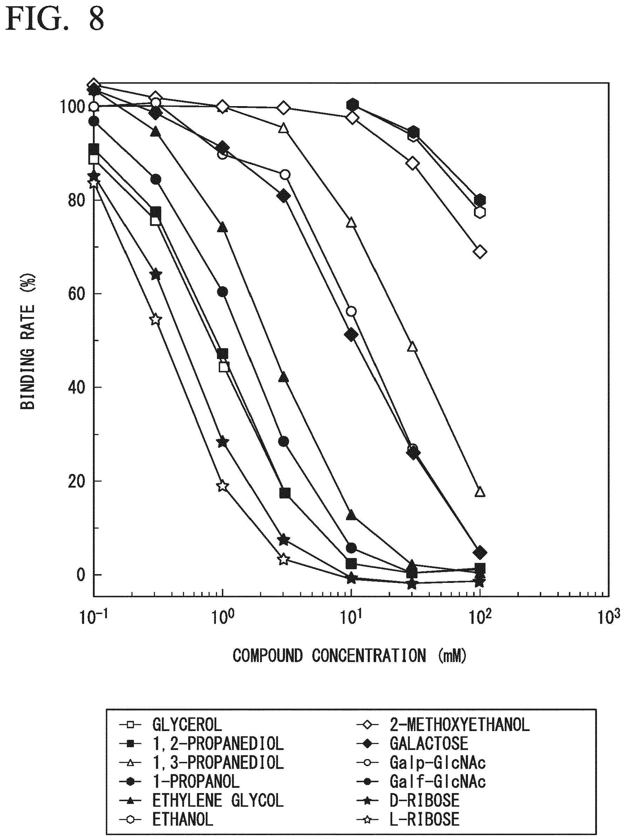

Figure 8 is a graph that shows an experiment result. Intelectin showed high affinities for compounds comprising a 1,2-diol group. Intelectin also showed low affinities for compounds comprising the 2,3-diol structure. Intelectin did not show almost any affinity for compounds not having a diol group.

The half maximal inhibitory concentrations (IC50) for inhibition of the binding of intelectin and the diol group by various compounds were calculated according to the measurement results using Biacore (GE Healthcare Biosciences). Table 1 shows the IC50 value for each compound.

**[Table 1]**

| Compound | IC₅₀ (mM) | Compound | IC₅₀ (mM) |
|---|---|---|---|
| L-ribose | 0.3 | D-galactose | 11 |
| D-ribose | 0.5 | Galp-GlcNAc | 13 |
| L-ascorbic acid | 0.8 | L-lyxose | 13 |
| monobutyrin | 0.8 | L-sorbose | 14 |
| glycerol | 0.8 | Tris | 14 |
| 1,2-butanediol | 0.8 | D-mannose | 16 |
| 1,2-propanediol | 0.9 | D-glucose | 18 |
| (R)-1,2-propanediol | 1.0 | D-lyxose | 22 |
| 2-deoxygalactose | 1.1 | L-arabinose | 22 |
| ribitol | 1.2 | 1,3-propanediol | 30 |
| alpha-glycerophosphate | 1.2 | L-rhamnose | 30 |
| (S)-1,2-propanediol | 1.2 | D-arabinose | 30 |
| Galf-GlcNAc | 1.4 | 1,3-butanediol | 35 |
| 3-amino-1,2-propanediol | 1.8 | N-acetylglucosamine | 50 |
| meso-2,3-butanediol | 1.8 | D-fucose | 60 |
| 2-deoxyribose | 1.8 | Ethanolamine | >100 |
| ethylene glycol | 2.2 | Beta-glycerophosphate | >100 |
| D-xylose | 3.3 | 2-methoxyethanol | >100 |
| (R,R)-2,3-butanediol | 5 | L-fucose | >100 |
| (S,S)-2,3-butanediol | 5 | 1,4-butanediol | >100 |
| 2-O-methyl-D-N-acetylneuraminic acid | 6 | ethanol | >100 |
| L-xyrose | 9 | 1-propanol | >100 |
| D-fructose | 9 | 3-amino-1-propanol | >100 |

### [Example 4]

### (Measurement of the dissociation constant between intelectin and a diol group)

Using Biacore (GE Healthcare Biosciences), the binding of purified intelectin to a sensor chip to which 3-amino-1-propanediol is fixed via an amino group was measured, and the dissociation constant was determined.

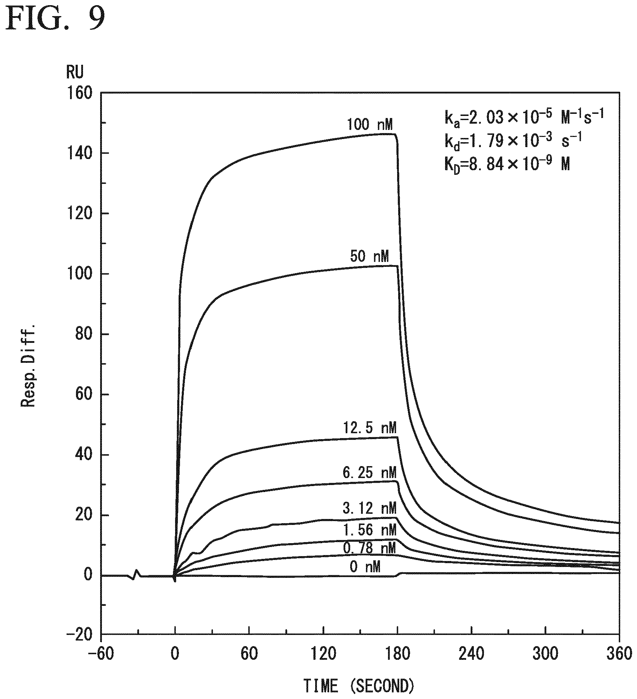

Figure 9 is a graph that shows a measurement result. Accordingly, an association rate constant Kₐ = 2.03 x10⁻⁵ M⁻¹ s⁻¹, a dissociation rate constant K_{d} = 1.79 x10⁻³ s⁻¹, and a dissociation constant K_{D} = 8.84 x10⁻⁹ M were obtained.

### [Example 5]

### (Binding of monoacylglycerol and intelectin)

Monoacylgrycerols such as monopalmitin and monoolein are used as a component of micelles or liposomes. 1-monopalmitin, 2-monopalmitin, or 1-palmitoyl lysophosphatidic acid as a negative control at the final concentration of 100 micro M was added to 500 microliters of a medium containing intelectin, and the medium was stirred intensely and incubated for 15 minutes at 37 deg C. Recombinant human intelectin-1 was used as intelectin. By the above-mentioned procedures, it is expected that insoluble aggregates having a micelle-like structure with the diol group facing outwards and the acyl group facing inwards are formed and intelectin binds on the surface.

Subsequently, the samples were cooled for 15 minutes at 4 deg C and centrifuged, and the precipitation was collected, washed once with buffer, and dissolved in sample buffer. Samples were then separated by SDS-PAGE and stained with Coomassie Brilliant Blue.

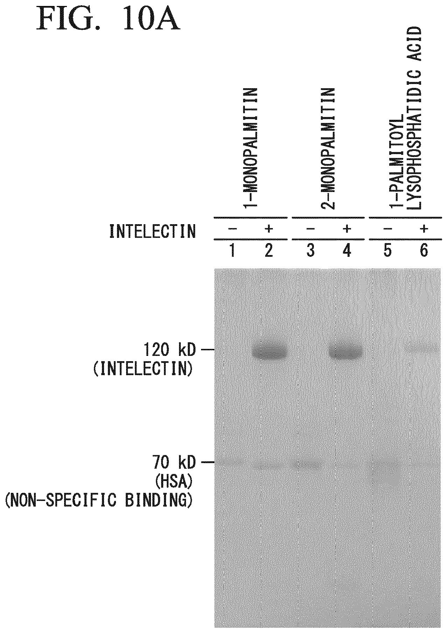

Figure 10A is a photograph that shows an SDS-PAGE result. Accordingly, intelectin was revealed to have bound to 1-monopalmitin and 2-monopalmitin. It was also shown that intelectin binds to 1-palmitoyl lysophosphatidic acid only slightly.

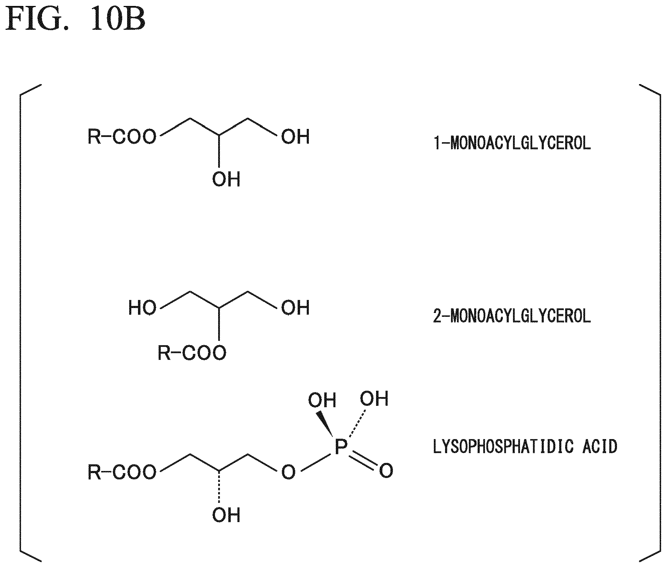

Figure 10B shows general formulae showing the structure of 1-monoacylglycerol represented by 1-monopalmitin, 2-monoacylglycerol represented by 2-monopalmitin, and lysophosphatidic acid represented by 1-palmitoyl lysophosphatidic acid. In Figure 10B, R represents an alkyl group.

### [Example 6]

### (Generation of a conjugate of an antibody Fab fragment and intelectin)

A nucleotide sequence fragment encoding a protein in which the H chain Fab region of the anti-interleukin (IL)-6 monoclonal antibody MH166 (the V_{H}-CHl region) is joined via one glycine residue as a spacer to the N terminus of the mature form of the mutant recombinant human intelectin-1 (S165A), in which ¹⁶⁵Ser is replaced with Ala and the N-glycosylation of ¹⁶³Asn is inhibited, was generated, and inserted into an expression plasmid.

The above-mentioned expression plasmid and an expression plasmid in which a nucleotide sequence fragment encoding the L chain of the monoclonal antibody MH166 is incorporated were introduced genetically into the rabbit kidney cell line RK-13, and the genes were co-expressed.

The nucleotide sequence encoding the mature protein form of the mutant recombinant human intelectin-1 (S165A) (including the spacer, one glycine residue) is shown in SEQ ID NO: 3. The nucleotide sequence encoding the H chain Fab region of MH 166 is shown in SEQ ID NO: 4, and the nucleotide sequence encoding the L chain of MH166 is shown in SEQ ID NO: 5.

Subsequently, the culture supernatant of the above-mentioned cell line was collected and added to diol Sepharose beads, and beads were washed. The sample was eluted with 20 mM Tris buffer (pH 7.4) containing 10% 1,2-propanediol, and 0.05% Tween 20. The elution fraction was added to the Resource Q column (1 mL, GE Healthcare Biosciences) and eluted by a NaCl concentration gradient of 0-1 M.

Each fraction was separated by SDS-PAGE under reducing conditions and stained with Coomassie Brilliant Blue, and the presence of the conjugate of the antibody Fab fragment and intelectin was confirmed.

### [Example 7]

### (Generation of a conjugate of TNFR2 and intelectin)

A nucleotide sequence fragment encoding a protein in which the extracellular region of human TNF receptor 2 (TNFR2) is joined via one glycine residue as a spacer to the N terminus of the mature form of the above-mentioned mutant recombinant human intelectin-1 (S165A) was generated, and inserted into an expression plasmid. The nucleotide sequence encoding the extracellular region of human TNFR2 used is shown in SEQ ID NO: 6. The above-mentioned plasmid was introduced genetically into the rabbit kidney cell line RK13, and the gene was expressed.

Subsequently, the culture supernatant of the above-mentioned cell line was collected and added to diol Sepharose beads, and beads were washed. The sample was eluted with 20 mM Tris buffer (pH 7.4) containing 10% 1,2-propanediol and 0.05% Tween 20. The elution fraction was added to the Resource Q column (1 mL, GE Healthcare Biosciences) and eluted by a NaCl concentration gradient of 0-1 M.

Each fraction was separated by SDS-PAGE under reducing conditions and stained with Coomassie Brilliant Blue, and the presence of the conjugate of TNFR2 and intelectin was confirmed.

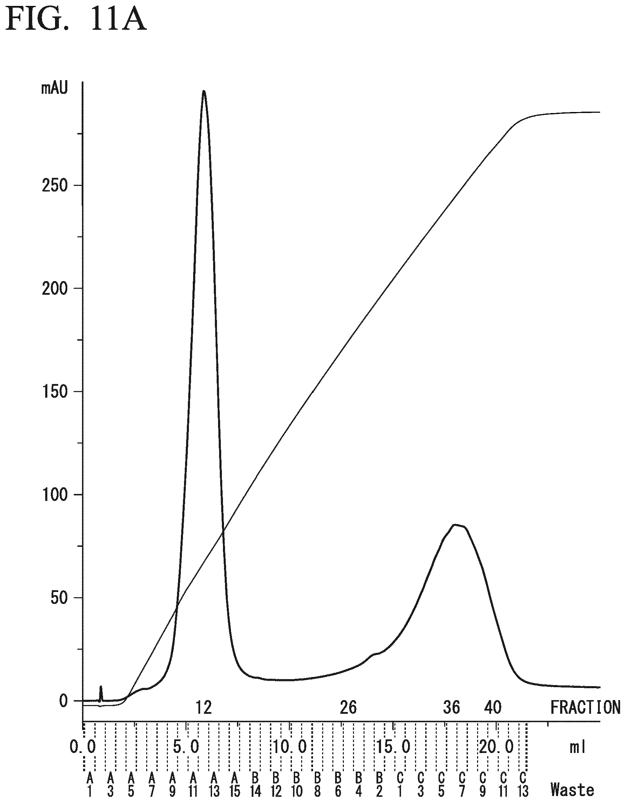

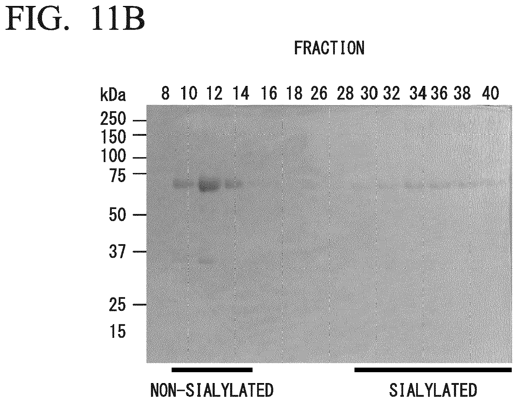

Figure 11A is a graph that shows an elution profile of the conjugate by anion-exchange chromatography. Figure 11B is a photograph that shows an SDS-PAGE result. Accordingly, the conjugate of TNFR2 and intelectin is shown to be able to be highly purified by diol Sepharose beads and anion-exchange chromatography.

### [Example 8]

### (Investigation of the reactivity of a conjugate of an antibody Fab fragment and intelectin)

An ELISA analysis on IL-6 protein was performed using the conjugate generated in Example 6.

More specifically, IL-6 protein was adsorbed on an ELISA plate, and the conjugate generated in Example 6 was used for reaction as the primary antibody, and the horseradish peroxidase-labeled anti-intelectin-1 monoclonal antibody (2D2) was used for reaction as the secondary antibody. The detection was conducted using 3,3',5,5'-tetramethylbenzidine.

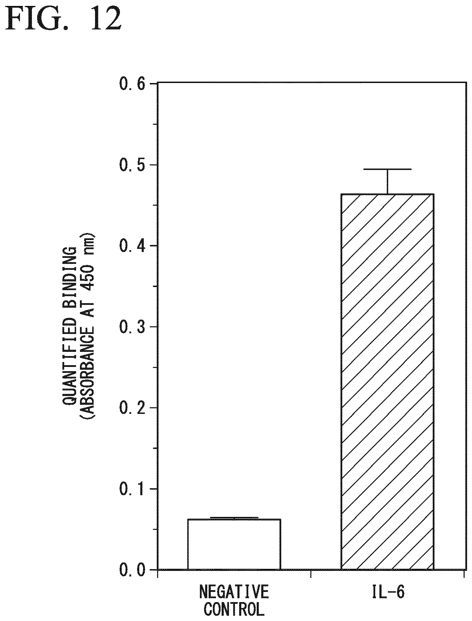

Figure 12 is a graph that shows an experiment result. Accordingly, the conjugate generated in Example 6 is now revealed to bind to IL-6 protein.

### [Example 9]

### (Investigation of the reactivity of a conjugate of TNFR2 and intelectin)

A test of adsorbing and removing TNF-alpha in solution was performed using the conjugate generated in Example 7. The conjugate generated in Example 7 was serially diluted, added to 50 microliters of a medium containing human TNF-alpha, and allowed to react for 30 minutes at room temperature. The complex of TNF-alpha and the conjugate were then adsorbed using 3 microliters of diol Sepharose beads. Subsequently, TNF-alpha in the supernatant was measured by ELISA, and the percentage of the remaining TNF-alpha was calculated. Experiments were done twice, independently.

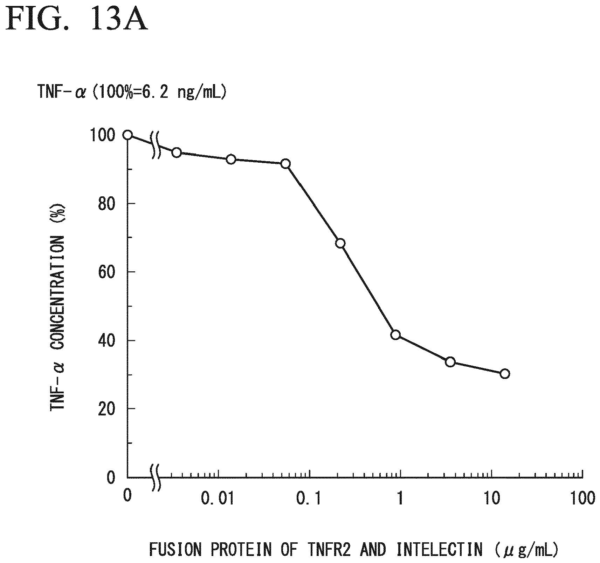

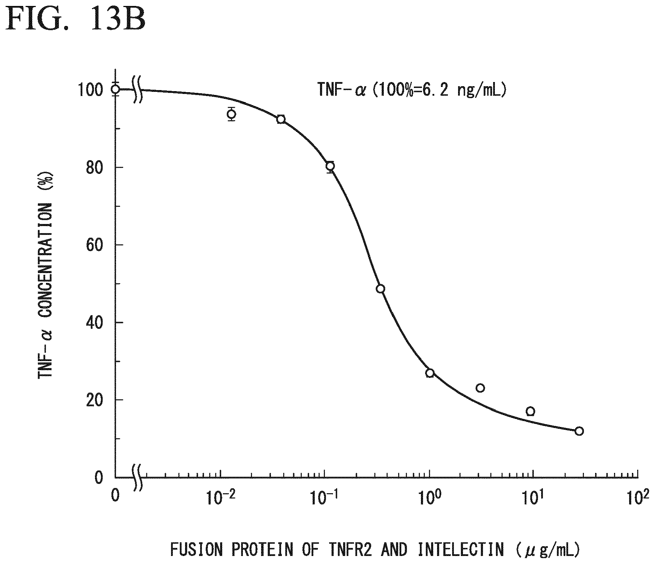

Figure 13A and B are the graphs that show the results from the 1st and the 2nd experiments, respectively. Accordingly, the conjugate generated in Example 7 is now revealed to be able to adsorb and remove TNF-alpha in solution.

### [Example 10]

### (Binding of mouse intelectin-1 and human intelectin-1 monomers to a diol group)

Beads having a diol group on the surface and beads not having a diol group on the surface were prepared. Diol beads, diol Sepharose brads, and hydroxy beads, that were used in Example 1, were used as the beads.

Wild type mouse intelectin-1 and mutant human intelectin-1 were used as intelectin.

Wild type mouse intelectin-1 is produced as a monomer. Mouse intelectin-1 has 51 amino acid residues that are different from human intelectin-1, at the amino acid level.

On the other hand, as described above, wild type human intelectin-1 forms a trimer. The mutant (C31, 48S), in which each of the cysteine residues at position 31 and position 48 are substituted with a serine residue, does not form a trimer bridged by disulfide bonds and is produced as a monomer. The amino acid sequence of the C31, 48S (C31A, C48S) mutant of human intelectin-1 is shown in SEQ ID NO: 9. The nucleotide sequence of the C31, 48S mutant of human intelectin-1 is shown in SEQ ID NO: 10.

First, beads were added to 5 mL of a medium containing recombinant mouse intelectin-1 or 1 mL of a medium containing C31, 48S mutant of human intelectin-1, and the samples were stirred for 18 hours at 25 deg C.

Subsequently, the beads were collected by centrifugation and washed once with buffer, and the samples were eluted with buffer containing 15% glycerol. The samples were then separated by SDS-polyacrylamide gel electrophoresis (SDS-PAGE), and proteins were stained with Coomassie Brilliant Blue.

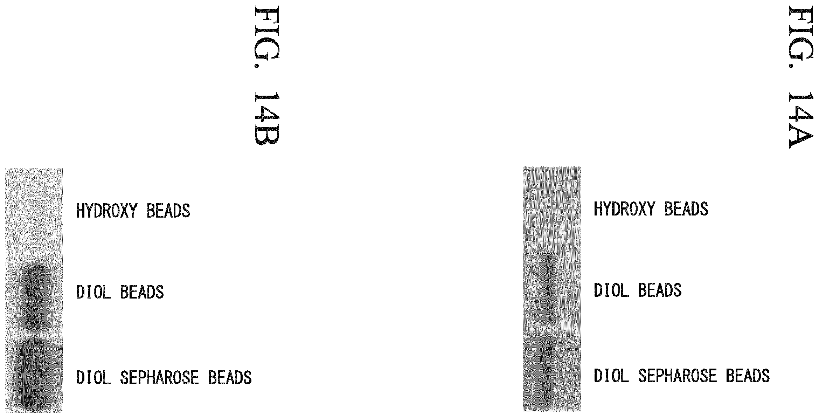

Figure 14A is a photograph that shows an SDS-PAGE result with mouse intelectin-1. Figure 14B is a photograph that shows an SDS-PAGE result with the C31, 48S mutant of human intelectin-1.

Accordingly, mouse intelectin-1 and the C31, 48S mutant of human intelectin-1 were revealed to specifically bind to diol beads and diol Sepharose beads as with human intelectin-1.

These results show that even when there are at least 51 mutations in the amino acid sequence shown in SEQ ID NO: 1 the binding activity to the diol group is present. These results also show that intelectin as a monomer also has the binding activity to the diol group.

### [Example 11]

### (Measurement of the affinity between the conjugate of TNFR2 and intelectin and TNF-alpha)

The binding affinity between the conjugate generated in Example 7 and TNF-alpha was measured using Biacore (GE Healthcare Biosciences). The binding affinity between a commercially available anti-TNF formulation (Etanercept, soluble TNF receptor, product name "Enbrel (registered trademark)," Takeda Pharmaceutical Company) and TNF-alpha was also measured as a control.

Recombinant human TNF-alpha (Life Technologies) was used as TNF-alpha. TNF-alpha was used as an analyte, and the conjugate generated in Example 7 or Etanercept was fixed to a sensor chip via an amino group and was used as the ligand. 10 mM HEPES (pH 7.0) containing 1 mM CaCl₂, 150 mM NaCl, and 0.03% Tween 20 was used as buffer for measuring affinities.

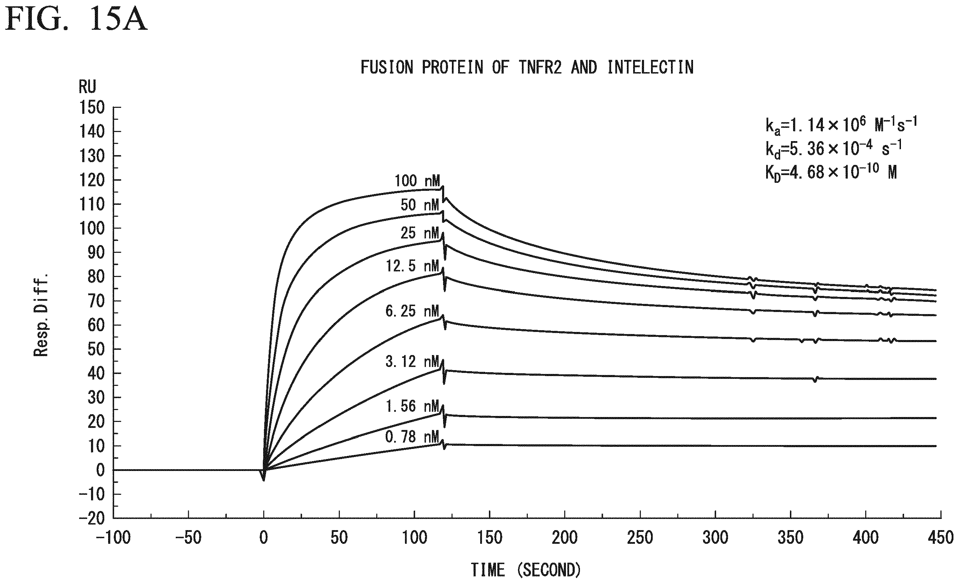

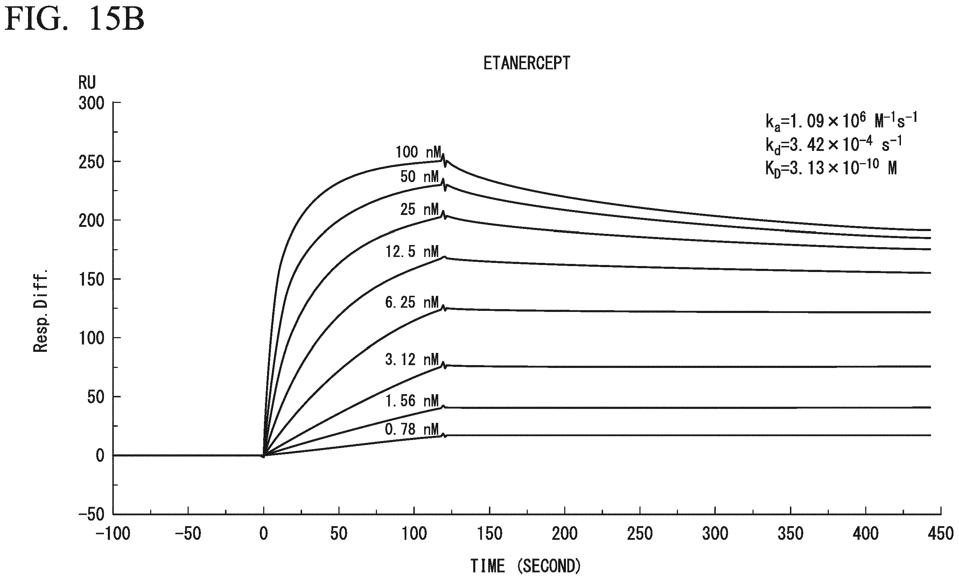

Figure 15A is a graph that shows a result from measuring the affinity between the conjugate generated in Example 7 and TNF-alpha. Figure 15B is a graph that shows a result from measuring the affinity between Etanercept and TNF-alpha.

Accordingly, the affinity between the conjugate generated in Example 7 and TNF-alpha was calculated as an association rate constant Kₐ = 1.14 x10⁶ M⁻¹ s⁻¹, a dissociation rate constant K_{d} = 5.36 x10⁻⁴ s⁻¹, and a dissociation constant K_{D} = 4.68 x10⁻¹⁰ M.

In contrast, the affinity between Etanercept and TNF-alpha was calculated as an association rate constant Kₐ = 1.09 x10⁶ M⁻¹ s⁻¹, a dissociation rate constant K_{d} = 3.42 x10⁻⁴ s⁻¹, and a dissociation constant K_{D} = 3.13 x10⁻¹⁰ M.

Based on the results above, the conjugate generated in Example 7 and Etanercept have approximately equivalent TNF-alpha binding activities.

### [Example 12]

### (Investigation of the inhibitory effect of the conjugate of TNFR2 and intelectin on cytotoxicity by TNF-alpha)

Actinomycin D at the final concentration of 2.12 micro M, recombinant human TNF-alpha (Life Technologies) at the final concentration of 1 ng/mL, and serially diluted conjugates generated in Example 7 were added to the medium of L929, a mouse-derived cell line, and the cells were cultured for 24 hours at 37 deg C. Subsequently, the number of live cells were measured using a commercially available kit (model "CellTiter96 (Trademark)," Promega), and the cell survival rate was determined. Another experiment, where a commercially available anti-TNF formulation (Etanercept, soluble TNF receptor, product name "Enbrel (registered trademark)," Takeda Pharmaceutical Company) was used instead of the conjugate generated in Example 7, was also performed as a control.

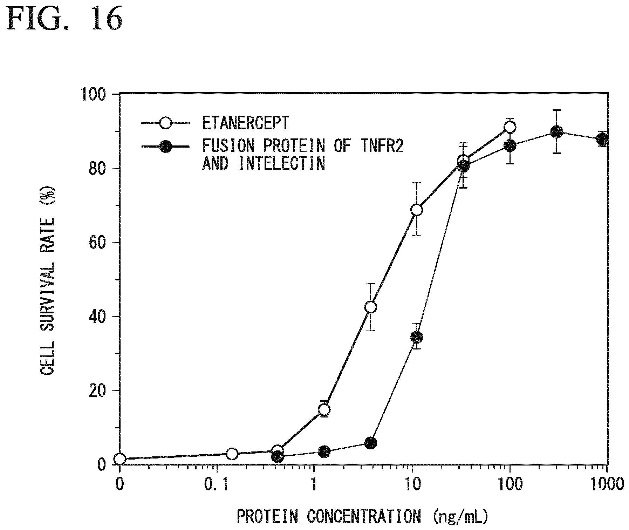

Figure 16 is a graph that shows an experiment result. Accordingly, the conjugate generated in Example 7 and Etanercept are now revealed to have approximately equivalent cytotoxicity inhibitory activities.

### [Example 13]

### (Investigation of the inhibitory effect of the conjugate of TNFR2 and intelectin on recombinant human TNF-alpha induced death in mice)

150 microliters of phosphate-buffered saline (PBS) containing 20 micrograms of the conjugate generated in Example 7 was administered via the tail vein to 7 week-old female C3H/HeN mice (10 mice per group), and this was used as the test group. 150 microliters of PBS only was administered via the tail vein to the mice in the control group.

Subsequently, 1 mL of PBS containing the mixture of 18 mg of galactosamine and 1.5 micrograms of recombinant human TNF-alpha (Life Technologies) was intraperitoneally administered and the survival was successively observed.

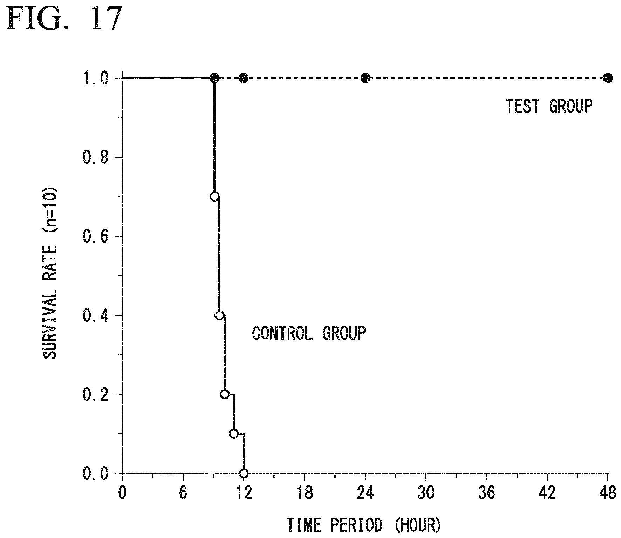

Figure 17 is a graph that shows an experiment result. Accordingly, since all the mice in the control group died within 12 hours, while no individuals in the test group became weak or died until 4 days later, the observation was terminated after 4 days.

Based on the above results, the conjugate generated in Example 7 is now revealed to be able to suppress TNF-alpha induced death in mice.

### [Example 14]

### (Adsorption of intelectin in human serum to diol modified silica gel)

40 microliters of 50% fresh human serum diluted with 0.1% Tween 20 -150 mM NaCl - 50 mM phosphate buffer (pH 7.3) added with or not added with 10 mM EDTA was added to a gel filtration chromatography column (TSKgel G3000 SWXL Tosoh Corporation) utilizing diol-modified silica gel as the carrier, and gel filtration was performed using 0.1% Tween 20 -150 mM NaCl - 50 mM phosphate buffer (pH 7.3) as solvent. Subsequently, the intelectin concentration in the elution fraction was measured using ELISA.

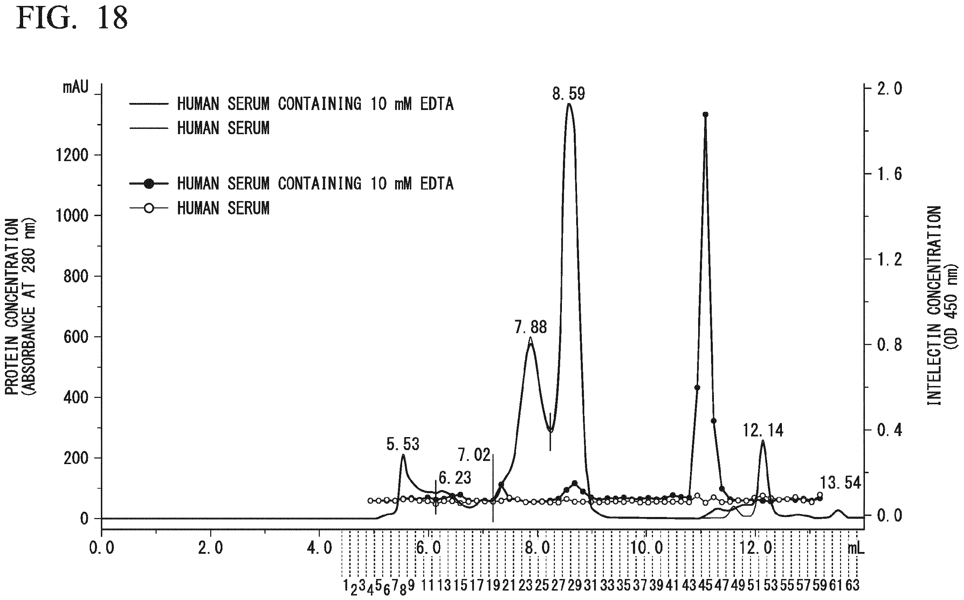

Figure 18 is a graph that shows an experiment result. Accordingly, in the serum sample added with 10 mM EDTA, intelectin did not bind to the column, and the intelectin peak was detected near the elution solution volume of around 11 mL (a peak of closed circle in the figure.). On the other hand, in the serum sample not added with EDTA, intelectin was not detected in any of the fractions (open circle), and intelectin was revealed to have been adsorbed to the column.

### INDUSTRIAL APPLICABILITY

According to the present invention, we are able to provide a technology that allows feasible purification of a biologically active protein, for example an antibody, at low cost. We are also able to provide a conjugate, a vector, a pharmaceutical composition, a pharmaceutical, a method of purifying a conjugate, a solid phase for capturing a conjugate, a method of removing a target substance, a device for removing a target substance, a labeling compound for detecting a conjugate, a method of detecting a conjugate, a conjugate for detecting a compound, and a method of detecting a compound.

### EXPLANATIONS OF REFERENCE NUMERALS

100 = pharmaceutical composition; 110 = drug; 120, 251 = compound; 130 = carrier; 140, 230, 430 = conjugate (removing means); 141 = intelectin or the mutant thereof; 142 = antibody; 300, 400 = device for removing a target substance; 210 = target substance, 220, 220' = plasma, 240 = complex; 250 = solid phase (removing means); 255 = column; 260 = blood circuit; 270a, 270b = pump; 280 = plasma separation device; 290 = plasma circuit.

## Claims

1. A conjugate of:
a protein consisting of the amino acid sequence of SEQ ID NO: 1, or
a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂.]; and
a functional molecule.

2. The conjugate of Claim 1, wherein said functional molecule is a target substance capturing molecule.

3. The conjugate of Claim 2, wherein said target substance capturing molecule is an antibody, an antibody fragment, an aptamer, or a receptor against said target substance.

4. The conjugate of Claim 2 or 3, wherein said target substance capturing molecule is tumor necrosis factor receptor (TNFR).

5. The conjugate of any one of Claims 2-4, wherein said target substance is a cytokine.

6. A vector **characterized by** containing a nucleic acid encoding a fusion protein of:
a protein consisting of the amino acid sequence of SEQ ID NO: 1, or
a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H₂]; and
an antibody, an antibody fragment, a peptide aptamer, or a receptor against a target substance.

7. A pharmaceutical composition **characterized by** comprising:
a drug;
a carrier containing a compound comprising -OH and -OR¹ [R¹ represents a hydrogen atom, an alkyl group, or -PO₃H_{2.}]; and
a conjugate of any one of Claims 1-5 bound to said compound.

8. A pharmaceutical **characterized by** containing a conjugate of any one of Claims 1-5 as an active ingredient.

9. A method of purifying a conjugate, the method **characterized by** comprising
contacting a conjugate of any one of Claims 1-5 with a compound comprising -OH and -OR¹ [R¹ represents a hydrogen atom, an alkyl group, or -PO₃H_{2.}] fixed to a solid phase.

10. A solid phase for capturing a conjugate of any one of Claims 1-5, the solid phase **characterized in that** a compound comprising -OH and -OR¹ [R¹ represents a hydrogen atom, an alkyl group, or -PO₃H_{2.}] is fixed to said solid phase.

11. A method of removing a conjugate of any one of Claims 1-5, the method **characterized by** comprising
contacting plasma containing said conjugate with a compound comprising - OH and -OR¹ [R¹ represents a hydrogen atom, an alkyl group, or -PO₃H_{2.}] fixed to a solid phase.

12. A method of removing a target substance, the method **characterized by** comprising contacting
plasma containing a complex of a target substance and a conjugate of any one of Claims 2-5 with
a compound comprising -OH and -OR¹ [R¹ represents a hydrogen atom, an alkyl group, or -PO₃H_{2.}] fixed to a solid phase.

13. A method of removing a target substance, the method **characterized by** comprising contacting
plasma containing a target substance with
a conjugate of any one of Claims 2-5 fixed to a solid phase.

14. A device for removing a conjugate of any one of Claims 1-5, the device **characterized by** comprising
a removing means for removing said conjugate from plasma containing said conjugate, wherein said removing means comprises a solid phase to which a compound comprising -OH and -OR¹ [R¹ represents a hydrogen atom, an alkyl group, or -PO₃H₂.] is fixed.

15. A device for removing a target substance, the device **characterized by** comprising
a removing means for removing a complex of a target substance and a conjugate of any one of Claims 2-5 from plasma containing said complex, wherein said removing means comprises a solid phase to which a compound comprising -OH and -OR¹ [R¹ represents a hydrogen atom, an alkyl group, or -PO₃H_{2.}] is fixed.

16. A device for removing a target substance, the device **characterized by** comprising
a removing means for removing a target substance from plasma containing said target substance, wherein said removing means comprises a solid phase to which a conjugate of any one of Claims 2-5 is fixed.

17. A labeling compound for detecting a conjugate of any one of Claims 1-5, the compound **characterized by** comprising -OH and -OR¹ [R¹ represents a hydrogen atom, an alkyl group, or -PO₃H₂.].

18. A method of detecting a conjugate, the method comprising
forming a complex by allowing a labeling compound comprising -OH and - OR¹ [R¹ represents a hydrogen atom, an alkyl group, or -PO₃H_{2.}] to bind to a conjugate of any one of Claims 1-5; and
detecting the labeling compound in said complex.

19. A conjugate for detecting a compound comprising -OH and -OR¹ [R¹ represents a hydrogen atom, an alkyl group, or -PO₃H₂.], the conjugate **characterized by** being a conjugate of:
a protein consisting of the amino acid sequence of SEQ ID NO: 1, or
a protein consisting of the amino acid sequence in which one or several amino acids have been deleted, substituted or added to the amino acid sequence of SEQ ID NO: 1, and having binding activity to a compound comprising -OH and -OR¹ [R¹ represents a hydrogen atom, an alkyl group, or -PO₃H_{2.}]; and
a labeling substance.

20. A method of detecting a compound comprising -OH and -OR¹ [R¹ represents a hydrogen atom, an alkyl group, or -PO₃H₂.], the method **characterized by** comprising
forming a complex by allowing a conjugate for detecting a compound of Claim 19 to bind to a compound comprising -OH and -OR¹ [R¹ represents a hydrogen atom, an alkyl group, or -PO₃H_{2.}]; and
detecting said labeling substance in said complex.
